# FASCICULE DE BREVET EUROPEEN

(11) **EP 2 201 062 B1**
(45) Date de publication et mention de la délivrance du brevet: **21.11.2012**
(21) Numéro de dépôt: 08805025.7
(22) Date de dépôt: 02.10.2008
(51) Int. Cl.: C08G 77/388, C08G 77/34

(54) **POLYORGANOSILOXANE A FONCTION PIPERIDINE DEPOURVU DE TOXICITE PAR CONTACT CUTANE, ET UTILISATION DE CE DERNIER DANS DES COMPOSITIONS COSMETIQUES**
POLYORGANOSILOXAN MIT PIPERIDINFUNKTION OHNE TOXIZITÄT BEI KONTAKT MIT DER HAUT UND VERWENDUNG DAVON IN KOSMETISCHEN ZUSAMMENSETZUNGEN
POLYORGANOSILOXANE WITH A PIPERIDINE FUNCTION, DEVOID OF TOXICITY UPON CONTACT WITH THE SKIN, AND USE THEREOF IN COSMETIC COMPOSITIONS

(30) Priorité: 02.10.2007 FR 0706892
(43) Date de publication de la demande: 30.06.2010
(73) Titulaire: Bluestar Silicones France, 69486 Lyon Cedex 03 (FR)
(72) Inventeur: LORENTZ, Gilles, F-69006 Lyon (FR); VIVIER, Richard, F-69003 Lyon (FR); RAMDANI, Kamel, F-69420 Tupin et Semons (FR); HENDRICKX, Bernard, F-01390 Tramoyes (FR)
(74) Mandataire: Mekki, Boualem
(86) Numéro de dépôt international: PCT/EP2008/063247
(87) Numéro de publication internationale: WO 2009/047212

(56) Documents cités:
- EP-A1- 1 726 294
- WO-A1-97/33034
- WO-A1-2004/097113
- US-A- 5 688 889

## Description

La présente invention est relative à de nouveaux polyorganosiloxanes comportant par molécule au moins un motif siloxyle substitué par au moins un groupement à fonction(s) pipéridinyle(s) stériquement encombrée(s) dépourvus de toxicité par contact cutané.

La présente invention concerne également une composition cosmétique améliorée comprenant les polyorganosiloxanes selon l'invention ainsi que l'utilisation de ces compositions pour le traitement des matières kératiniques, en particulier la peau et les cheveux.

De nombreuses compositions cosmétiques comprennent des polyorganosiloxanes («silicones»). Les polyorganosiloxanes peuvent être utilisés pour procurer un effet de conditionnement des cheveux ou de la peau. Les polyorganosiloxanes peuvent également être utilisés pour des effets sensoriels, dits effets cosmétiques, lors de leur application sur la peau, les cheveux ou les lèvres.

Ainsi, il a été proposé d'utiliser dans des compostions cosmétiques des polyorganosiloxanes de nombreuses structures chimiques différentes, pouvant éventuellement porter différents groupes fonctionnels.

Les polydiméthylorganosiloxanes (PDMS) linéaires peuvent être utilisés comme agents sensoriels sur la peau, comme agents protecteurs faisant barrière à l'eau, comme agents démoussants, comme agents éliminateurs de blancheurs crémeuses de compositions cosmétiques apparaissant lors d'un premier frottement sur la peau ou les cheveux «desoapers», comme agents conditionneurs ou comme agents émollients.

Les documents WO 97/33034 et US 5,688,689 décrivent des polvorganosiloxanes comportant des motifs siloxyles substitués par des groupements pipéridinyles stériquement encombrés.

On a en outre décrit des compositions cosmétiques comprenant des polyorganosiloxanes aminés, souvent en liaison avec des bénéfices en matière de coloration. La présence des groupes aminés améliore l'affinité du polyorganosiloxane pour les cheveux et procure un bon conditionnement des cheveux, pour des compositions destinées à être rincées ou à ne pas être rincées.

Par exemple, les documents US 6,605,577 (Chemsil Silicones Inc.), US 6,642,194 (Chemsil Silicones Inc.), WO 03/088939 (The Procter & Gamble Company), WO 03/066007 (Dow Corning), EP 1 726 294 (L'Oréal) WO 2004/097113 (Rhône Poulenc Chimie) décrivent des compositions cosmétiques comprenant un polyorganosiloxane portant des groupes fonctionnels particuliers.

On entendra dans toute la description par matières kératiniques, les matières à traiter cosmétiquement ou dermatologiquement choisies parmi la peau, le cuir chevelu, les cheveux, les cils, les sourcils, les ongles ou les muqueuses.

On entendra dans toute la description par produit cosmétique ou dermatologique «non-rincé», tout produit dont l'application sur les matières kératiniques à traiter n'est pas suivie d'un rinçage à l'eau.

On entendra dans toute la description par produit cosmétique ou dermatologique «rincé», tout produit dont l'application sur les matières kératiniques à traiter est suivie d'un rinçage à l'eau.

De nombreuses compositions de lavage des matières kératiniques ont été décrites dans l'art antérieur. Ainsi, la demande de brevet FR-A-2 848 829 décrit des compositions détergentes destinées essentiellement au lavage des cheveux, qui présentent des propriétés cosmétiques améliorées en particulier en termes de démêlage, lissage, souplesse, maléabilité et douceur des cheveux. Ces compositions comprennent au moins un tensioactif amphotère alcoylamphohydroxyalkylsulfonate, et au moins une silicone choisie parmi les silicones non organomodifiées de viscosité allant de 500 mm²/s à 1 000 000 mm²/s et les silicones organomodifiées.

La demande de brevet CA-1 201 952 décrit des compositions tensioactives destinées à la formulation de compositions cosmétiques, en particulier des shampooings, dont le pouvoir moussant est amélioré. Ces compositions comprennent:
- de 3,75 à 15 % en poids d'un tensioactif anionique tel qu'un lauryl éther sulfate,
- de 1 à 4,20 % en poids d'un tensioactif amphotère tel qu'une alkyl amidobétaïne,
- de 0,7 à 3% en poids d'un tensioactif non ionique tel qu'un sorbitan polyoxyéthyléné, et
- de 0,1 à 4 % en poids d'un « savon » choisi parmi les acides gras, les alkyle isethionates, les alkyles taurides et les sarcosides d'alkyles.

La demande de brevet US 2004/0197287 décrit des shampooings antipelliculaires dont les propriétés détergentes et antipelliculaires sont améliorées. Ces compositions comprennent :
- de 5 à 50% en poids d'un tensioactif détergent,
- de 0,1 à 4% en poids d'un agent antipelliculaire,
- de 0,1 à 50% en poids d'un ester d'acide gras et

La demande de brevet WO 97/33561 décrit des compositions nettoyantes pour les cheveux et la peau, qui présentent un faible degré d'irritation oculaire. Ces compositions comprennent :
- de 5 à 20 % en poids d'un mélange de tensioactifs dont un tensioactif non ionique, un tensioactif amphotère et un tensioactif anionique, et
- de 0,01 à 3% en poids d'un agent humectant, tel que notamment un polyol cationique.

Enfin, la demande de brevet FR-A-2 886 145 décrit des compositions de lavage des matières kératiniques, en particulier des cheveux comprenant, dans un milieu aqueux cosmétiquement acceptable, au moins un tensioactif détergent anionique, non ionique ou amphotère, au moins un polymère cationique et au moins une silicone aminée comprenant une fonction amine portée par un groupe stériquement encombré. Ces composition permettent d'améliorer les propriétés de lissage visuel et au toucher des cheveux, et ceci tout en ayant un bon pouvoir lavant et moussant.

Il est connu dans l'art antérieur que certains composants de produits cosmétiques ou d'hygiène, comme les conservateurs chimiques, les parfums, les colorants, les filtres solaires chimiques, l'éthanol, etc. peuvent causer des problèmes d'irritation et/ou d'intolérance cutanée, voir des problèmes d'allergie de contact. Les peaux sensibles ou réactives sont des peaux qui réagissent facilement aux allergènes ou irritants suite à des troubles de la perméabilité cutanée liés à l'altération de la fonction barrière du stratum cornéum et d'un déséquilibre de la production des cytokines épidermiques. La modification de la perméabilité cutanée donne lieu à l'apparition de signes subjectifs et objectifs.

L'apparition des signes subjectifs est particulièrement observée lors de l'utilisation de produits cosmétiques ou d'hygiène courant. Ils se définissent par des sensations de démangeaisons, tiraillements, picotements, chaleurs, piqûres et brûlures. Les signes objectifs se montrent de façon irrégulière par une xèrose, par une dermite séborrhéique, par des télangiectasies, par des squames, par une rougeur, par des vésicules ou même par un oedème.

Selon les spécialistes, les signes subjectifs et objectifs peuvent apparaître pour une courte durée immédiatement après l'application du produit cosmétique, ou peuvent être signalés de façon transitoire, ou bien peuvent persister plus longtemps, toute la journée ou de façon intermittente au cours de la journée. Ainsi ces signes peuvent être discrets ou sévères et peuvent nécessiter un avis médical.

Ainsi, les compositions décrites dans l'art antérieur peuvent présenter certaines insuffisances. En particulier, les shampooings les plus performants peuvent provoquer des picotements dans l'oeil lorsque le produit dilué coule dans la sphère oculaire, ce qui arrive fréquemment chez les enfants. Par ailleurs, bon nombre de ces shampooings peuvent provoquer, chez les personnes présentant une peau sensible, des réactions d'inconfort telles que des rougeurs, des démangeaisons ou des picotements. Cela peut-être le cas pour certaines silicones aminées comprenant une fonction amine portée par un groupe stériquement encombré et décrites par exemple dans la demande de brevet FR-A-2 886 145. Ces silicones aminées, plus communément appelées "silicones HALS", peuvent présenter des problèmes de sensibilisation cutanée et sont classées sensibilisantes "R43". Or, les matières premières cosmétiques présentant un caractère sensibilisant de la peau sont restreintes à des usages très limités suivant les pays (voir par exemple pour l'Europe: la Directive Cosmétique Européenne n°76/768/EEC).

Par exemple, en Europe, selon les critères de l'Annexe VI de la directive 67/548/CEE en sa 28ème adaptation au progrès technique (2001/59/CE), une substance ou une préparation chimique est classée comme sensibilisant cutané "R43" lorsqu'elle peut entraîner une sensibilisation par contact avec la peau. Cette sensibilisation par contact avec la peau doit avoir été observée chez un nombre significatif de personnes ou via un test approprié sur animaux.

Il existe ainsi plusieurs méthodes d'essai utilisant des cobayes: les tests de Magnusson & Kligman ou de Buehler. Récemment, le test "Local Lymph Node Assay" (LLNA) a été validé et adopté par la communauté scientifique et repris sous forme de ligne directrice OCDE 429 ou de méthode décrite dans l'annexe V de la directive 67/548/CEE en sa 29ème adaptation au progrès technique (2005/73/EC). Ce test est réalisé sur souris et se base sur l'induction de la prolifération de lymphocytes dans les ganglions sous-jacents au site d'application de la substance. Les résultats déterminent si la matière première testée doit être classée sensibilisante R43.

La Demanderesse a maintenant découvert, de manière surprenante, de nouveaux polyorganosiloxanes comportant par molécule au moins un motif siloxyle substitué par au moins un groupement à fonction(s) pipéridinyle(s) stériquement encombrée(s) dépourvus de toxicité par contact cutané et qui formulés dans des compositions cosmétiques particulièrement douces et non agressives, présentent de bonnes qualités d'usage et d'excellentes propriétés tant détergentes que cosmétiques.

La présente invention a donc pour premier objet des polyorganosiloxanes **A** dépourvus de toxicité par contact cutané comportant par molécule au moins un motif siloxyle substitué par au moins un groupement fonctionnel **V** comprenant au moins une fonction pipéridinyle stériquement encombrée lesdits polyorganosiloxanes **A** étant caractérisés en ce qu'après leur préparation on réduit le taux des monomères, oligomères et polymères ayant une masse moléculaire M <1000 Daltons à une valeur inférieure à 1% par rapport à la distribution moléculaire totale au moyen d'une technique permettant la séparation sélective des monomères, oligomères et polymères ayant une masse moléculaire **M** < 1000 Daltons (avec 1 Dalton = 1 g/mol).

Ainsi, les nouveaux polyorganosiloxanes **A** selon l'invention présentent l'avantage, dès lors que l'on réduit le taux des monomères, oligomères et polymères avant une masse moléculaire M <1000 Daltons à une valeur inférieure à 1% car rapport à la distribution moléculaire totale, de ne plus être classés "sensibilisant R43" et peuvent donc être utilisés dans le domaine de la cosmétique sans restriction. En effet, lorsqu'ils sont formulés dans des compositions cosmétiques, celles-ci ne présentent donc plus les réactions d'inconfort au niveau de la peau et du cuir chevelu, et possèdent un excellent niveau de tolérance oculaire. Parallèlement, elles possèdent des propriétés nettoyantes d'un bon niveau ainsi que d'excellentes propriétés cosmétiques, notamment un bon niveau de douceur en ce qui concerne les matières kératiniques telles que la peau, le cuir chevelu, les cheveux, les cils, les sourcils, les ongles ou les muqueuses peaux, les cheveux, et outre ce bon niveau de douceur, de bonnes propriétés de démêlage et/ou de lissage lorsqu'il s'agit de cheveux.

La séparation sélective des monomères, oligomères et polymères ayant une masse moléculaire **M** < 1000 Daltons dans un mélange d'huiles silicones peut se faire par exemple au moyen d'une technique dénommée "dévolatilisation". Cette technique est généralement utilisée dans le domaine de la purification des polymères. En effet, dans un procédé de fabrication d'un polymère silicone, la réaction de polymérisation devrait en théorie être complète et convertir la totalité du monomère en polymère. En réalité, on sait qu'une réaction de polymérisation n'est jamais complète, en particulier dans un procédé de polymérisation en solution ou en masse, en raison notamment de l'augmentation de la viscosité du milieu de polymérisation au cours de la réaction. C'est ainsi qu'en pratique, le polymère obtenu contient généralement des composés volatils résiduels tels que le monomère n'ayant pas réagi, un ou plusieurs solvants ajoutés ou accumulés pendant dans la réaction, et des oligomères formés au cours de la réaction. Cette technique consiste en une opération de dégazage du polymère. Elle est réalisée en soumettant le polymère chaud, en particulier sous forme d'une solution ou d'une masse fondue, à une pression réduite, de préférence une pression inférieure à la pression atmosphérique (ou pression sous-atmosphérique), dans une ou plusieurs chambres de détente, encore appelées « dévolatiliseurs » (en anglais: « flash tanks » ou « devolatilizers »), disposées en série et sous des vides successivement plus poussés. En particulier, le polymère peut être extrudé dans une chambre de détente sous la forme d'une masse fondue et divisée, par exemple sous la forme de « fils tombants » (en anglais « falling strands »), afin de faciliter la séparation entre les composés volatils résiduels et le polymère qui est ainsi récupéré, débarrassé de ces composés. De tels procédés de dégazage sont décrits par exemple dans les brevets américains US 2,970, 089, US 3,853,672, US 3,928,300, US 4,294,652, US 4,383,972, US 5, 453, 158, US 5,540,813 et US 5,874,525.

Lors de la préparation de polyorganosiloxanes fonctionnalisés par des groupements pipéridinyles stériquement encombrées, il est généralement effectué une étape de dévolatilisation standard (température comprise entre 150 et 200 °C, pression comprise entre 3 et 10 mbars pendant 1 à 2 h) et le taux des oligomères ayant une masse moléculaire M < 1000 Daltons est toujours supérieur à 1% de la distribution moléculaire totale (généralement ce taux est de l'ordre de 2 à 2,5 % de la distribution moléculaire totale).

Ainsi, tous les polyorganosiloxanes fonctionnalisés par des groupements pipéridinyles stériquement encombrées (par exemple des "silicones HALS") disponibles sur le marché présentent toujours un taux d'oligomères avant une masse moléculaire M < 1000 Daltons supérieur à 1,5% de la distribution moléculaire totale (généralement ce taux est de l'ordre de 2 à 2,5 % de la distribution moléculaire totale).

Il est important de comprendre qu'au sens de l'invention, l'homme du métier continuera l'étape de dévolatilisation suffisamment longtemps (par exemple pour des conditions standards de température et de pression: température comprise entre 150 et 200°C et pression comprise entre 3 et 10 mbars, le temps de dévolatilisation sera de 3 à 9 h au lieu de 1 à 2 h pour une dévolatilisation standard). Le temps de dévolatilisation sera déterminé suivant les conditions opératoires (température, pression,...) de manière à ce que jusqu'à ce que moins de 1% de la distribution moléculaire totale possède une masse moléculaire M < 1000 Daltons, la détermination du taux pouvant se faire par les techniques de mesures usuelles telles que par exemple la chromatographie par perméation de gel (GPC).

Selon une première variante, on peut aussi additionner un agent inerte et volatil au polymère. Cet agent est généralement connu sous le terme d'agent moussant ou gonflant, ou d'agent d'extraction (en anglais « foaming agent », « blowing agent » « stripping agent »), ou encore de fluide d'assistance ou d'aide à la dévolatilisation (en anglais « devolatilisation-assisting fluid » ou « devolatilisation aid »). Dans ces conditions, le dégazage du polymère consiste à détendre le mélange résultant de cette addition, à chaud et sous une pression réduite, dans une chambre de détente telle que celles décrites précédemment. Il résulte de cette détente que l'agent d'extraction ("stripping agent") forme un grand nombre de bulles à l'intérieur du polymère fondu, et que l'extraction par diffusion des composés volatils résiduels contenus dans le polymère est facilitée par la surface considérablement accrue de la masse moussante résultante. Des exemples de « stripping agent » sont décrits dans les brevets américains US 3,668,161, US 3,773,740, US 4, 195,169, US 4,537,954, US 5,350,813, US 5, 380,822 et 20 US 6,410,683. Ce sont généralement des fluides liquides dans les conditions normales et facilement volatils dans les conditions de dégazage, comme par exemple l'eau, les alcools ou les cétones, ou une solution de dioxyde de carbone dans l'eau. Dans les brevets américains US 5,691,445 et US 5,861,474, on a proposé de remplacer ces « stripping agent » conventionnels par un fluide super critique qui serait normalement gazeux dans les conditions d'injection, mais qui est maintenu en solution dans le polymère grâce à de hautes pressions appliquées pendant l'injection. Ainsi, on a par exemple proposé d'utiliser l'azote, le dioxyde de carbone et des alcanes notamment de C₄ à C₆.

Dans le domaine des silicones, selon la viscosité des produits il est possible de mettre en oeuvre différents appareillages et procédés pour la séparation sélective des composants à éliminer.

Selon un mode de réalisation préféré, la réduction du taux des monomères, oligomères et polymères ayant une masse moléculaire M < 1000 Daltons s'effectue au moyen d'un réacteur batch, d'un évaporateur à film tombant, d'un évaporateur à film raclé, d'un évaporateur centrifuge ou d'un évaporateur continu avec flash.

Un réacteur batch est un réacteur agité mécaniquement par un mobile par exemple une ancre, un ruban, des hélices et/ou des turbines contenant la masse liquide à purifier et est chauffé par sa paroi extérieure (double enveloppe ou coquille). Des échangeurs de chaleur peuvent être rajoutés dans le réacteur, par exemple sous la forme de serpentins. Un dispositif distributeur de gaz approprié peut permettre l'injection de gaz incondensable. Le dôme du réacteur est équipé d'un condenseur et relié à une pompe à vide. L'opération consiste à chauffer, diminuer éventuellement la pression et condenser les volatils au fur et à mesure qu'ils s'évaporent de la masse liquide. Plus la durée opératoire est longue et plus le produit est purifié.

Les évaporateurs à film tombant sont adaptés pour des produits ayant une viscosité moyenne. Le fluide est coulé de façon continue sur une paroi chauffée de manière à générer une couche de faible épaisseur que l'on appelle *film.* L'avantage du film est que la vapeur qui se forme traverse d'autant plus aisément la couche liquide que celle ci est mince. Le film progresse le long de la paroi de l'appareil sous l'effet de son propre poids, générant un brassage du film favorable au transfert de la chaleur et au transport des volatils vers la phase gaz. Le liquide s'écoule généralement de haut en bas où, purifié, il est collecté. Les évaporateurs à film tombant sont généralement constitués de tubes verticaux, éventuellement associés en grand nombre. Un dispositif collecte les vapeurs et les conduit au condenseur relié au circuit de vide. Plusieurs passages sur cet appareil, ou l'association de plusieurs appareils en série sont parfois requis pour atteindre une pureté donnée. Un balayage par un gaz incondensable est possible dans ces appareils.

Pour les évaporateurs à film raclé et pour accroitre l'efficacité du brassage et travailler avec des produits éventuellement plus visqueux, le film formé en continu sur un cylindre chauffé peut être agité par un système rotatif. Un rotor muni de racleurs qui balayent toute la surface du cylindre de manière à accroitre le brassage du film favorable au transfert de la chaleur et au transport des volatils vers la phase gaz. Le liquide s'écoule généralement de haut en bas soit par effet de son propre poids, soit grâce à la géométrie des racleurs qui ont un effet de "vis d'Archimède" faisant progresser le film vers le bas où, purifié, il est collecté. Un balayage par un gaz incondensable est possible dans ces appareils. Plusieurs passages sur cet appareil, ou l'association de plusieurs appareils en série sont parfois requis pour atteindre une pureté donnée.

Les évaporateurs centrifuges sont basés sur les mêmes principes, ces appareils sont alimentés en liquide au centre d'un rotor constitué de plaques d'échange de chaleur. L'accélération donnée au fluide par le mouvement rotatif du rotor le propulse vers l'extérieur. Les vapeurs qui se dégagent sont collectées dans une zone de désengagement appropriée et mises en contact avec le condenseur relié au circuit de vide.

Pour les évaporateurs continus avec flash, le fluide est alimenté en continu dans un système de type échangeur de chaleur, par exemple un corps de chauffe cylindrique à double enveloppe. Pour renouveler le fluide à la surface d'échange de chaleur, le corps de chauffe est muni d'internes, par exemple des mélangeurs statiques. Le fluide chaud arrive éventuellement sous pression dans un pot de détente sous pression réduite. Les volatils se vaporisent tandis que les liquides s'écoulent vers un collecteur. Les vapeurs sont condensées dans un échangeur et collectées à part.

Selon un mode préférentiel de l'invention, la réduction du taux des monomères, oligomères et polymères ayant une masse moléculaire M < 1000 Daltons s'effectue par une dévolatilisation poussée sous une pression réduite comprise entre 3 et 20 mbar et à une température comprise entre 100 et 210°C jusqu'à ce que moins de 1% de la distribution moléculaire totale possède une masse moléculaire M < 1000 Daltons.

Par "dévolatilisation poussée", il est entendu que la dévolatilisation est maintenue jusqu'à ce que le taux des monomères, oligomères et polymères ayant une masse moléculaire M < 1000 Daltons soit inférieur à 1% de la distribution moléculaire totale. Par exemple, pour une pression réduite de 4 mbar environ, le temps de "dévolatilisation poussée" sera de 3 à 9h au lieu de 1 à 2h pour une "dévolatilisation standard".

Selon un autre mode de réalisation préféré, les polyorganosiloxanes **A** dépourvus de toxicité par contact cutané selon l'invention sont caractérisés en ce qu'ils comportent par molécule au moins un motif siloxyle substitué par au moins un groupement fonctionnel **V** directement lié à un atome de silicium, ledit groupement fonctionnel **V** étant un groupement à fonction(s) pipéridinyle(s) stériquement encombrée(s) choisi parmi le groupe constitué par:
a) des groupements de formule **(I)** : 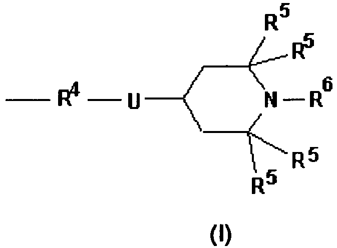 formule dans laquelle:
   - Les radicaux R⁵, identiques ou différents, sont choisis parmi les radicaux alkyles linéaires ou ramifiés ayant 1 à 3 atomes de carbone et le radical phényle; et le radical R⁶ représente un radical hydrogène, le radical R⁵ ou l'atome O*;
   - R⁴ est un radical hydrocarboné divalent choisi parmi le groupe constitué par :
      - les radicaux alkylènes linéaires ou ramifiés, ayant 2 à 18 atomes de carbone;
      - les radicaux alkylène-carbonyle dont la partie alkylène linéaire ou ramifiée, comporte 2 à 20 atomes de carbone;
      - les radicaux alkylène-cyclohexylène dont la partie alkylène linéaire ou ramifiée, comporte 2 à 12 atomes de carbone et la partie cyclo-hexylène comporte un groupement OH et éventuellement 1 ou 2 radicaux alkyles ayant 1 à 4 atomes de carbone;
      - les radicaux de formule R⁷-O-R⁷ dans laquelle les radicaux R⁷ identiques ou différents représentent des radicaux alkylènes ayant 1 à 12 atomes de carbone;
      - les radicaux de formule R⁷-O-R⁷ dans laquelle les radicaux R⁷ ont les significations indiquées précédemment et l'un d'entre eux ou les deux sont substitués par un ou deux groupement(s) -OH;
      - les radicaux de formule R⁷-COO-R⁷ dans laquelle les radicaux R⁷ ont les significations indiquées précédemment; et
      - les radicaux de formule R⁸-O-R⁹-O-CO-R⁸ dans laquelle les radicaux R⁸ et R⁹ identiques ou différents, représentent des radicaux alkylènes ayant 2 à 12 atomes de carbone et le radical R⁹ est éventuellement substitué par un radical hydroxyle; et
   - U représente -O- ou -N(R¹⁰)-, avec R¹⁰ étant un radical choisi parmi le groupe constitué par un atome d'hydrogène, un radical alkyle linéaire ou ramifié comportant 1 à 6 atomes de carbone et un radical divalent de formule **(II)** suivante : 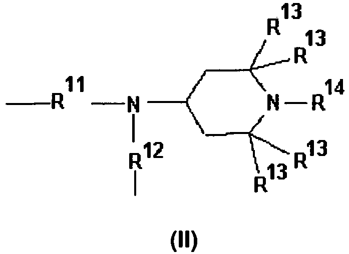
   dans laquelle R¹² a la même signification que pour R⁴ indiqué précédemment, R¹¹ représente un radical divalent alkylène, linéaire ou ramifié, ayant de 1 à 12 atomes de carbone, l'un des liens valentiels (celui de R¹¹) étant relié à l'atome de -NR¹⁰-, l'autre (celui de R¹²) étant relié à un atome de silicium; les radicaux R¹³ sont identiques ou différents, choisis parmi les radicaux alkyles linéaires ou ramifiés ayant 1 à 3 atomes de carbone et le radical phényle; et le radical R¹⁴ représente un radical hydrogène, le radical R¹³ ou l'atome O* ; et
b) des groupements de formule **(III):** 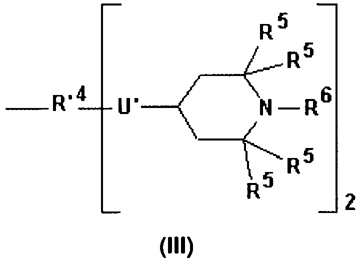 formule dans laquelle:
   R'⁴ est choisi parmi le groupe constitué par :
      - un radical trivalent de formule **(IV)** suivante: 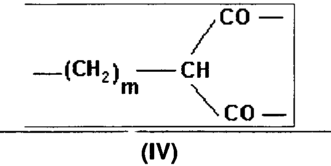
   où m représente un nombre de 2 à 20, et
   - un radical trivalent de formule **(VI) :** 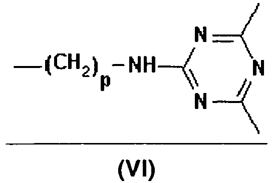
   où p représente un nombre de 2 à 20;
   - U' représente -O- ou -N(R¹²)-, avec R¹² étant un radical choisi parmi le groupe constitué par un atome d'hydrogène et un radical alkyle linéaire ou ramifié comportant 1 à 6 atomes de carbone; et
   - R⁵ et R⁶ ont les mêmes significations que celles données à propos de la formule **(I);** lesdits polyorganosiloxanes **A** sont caractérisés en ce qu'après leurs préparations on réduit le taux des monomères, oligomères et polymères ayant une masse moléculaire M < 1000 Daltons à une valeur inférieure à 1% par rapport à la distribution moléculaire totale au moyen d'une technique permettant la séparation sélective des monomères, oligomères et polymères ayant une masse moléculaire M < 1000 Daltons.

Les polyorganosiloxanes comportant par molécule au moins un motif siloxyle substitué par au moins un groupement fonctionnel **V**, tel que par exemple un groupement à fonction(s) pipéridinyle(s) stériquement encombrée(s), mais avec un taux de monomères, oligomères et polymères ayant une masse moléculaire M < 1000 Daltons supérieur à 1,5 % (en général, ils présentent un taux compris entre 2 et 2,5 %) par rapport à la distribution moléculaire totale sont bien connus. Par exemple, on peut citer ceux fournis par la société Bluestar Silicones sous la dénomination RHODORSIL^{®}Huile 21645, huile Hydrosoft^{®} ou RHODORSIL^{®} Huile 21650, ou ceux qui sont préparés suivant le mode opératoire décrit dans le brevet EP-0665258 ou dans le brevet US-5,721,297. On cite notamment les procédés comprenant un greffage du groupement fonctionnel V par une réaction d'hydrosilylation.

On cite également les procédés de redistribution ou re-arrangement à partir de polyorganosiloxanes ne comprenant pas le groupement fonctionnel **V**, et de polyorganosiloxanes comprenant des groupes groupement fonctionnel V liés à des atomes de silicium. Par exemple, un procédé adapté de redistribution ou re-arrangement peut comprendre les étapes suivantes:
1. Mettre en présence un polydiméthylsiloxane, de préférence cyclique, de l'hexaméthyldisiloxane, et un polyméthylsiloxane cyclique comprenant des groupements fonctionnels **V** liés à de atomes de silicium;
2. chauffer, introduire un catalyseur basique, par exemple une base forte, et laisser réagir; et
3. neutraliser; par exemple à l'aide d'un acide faible, puis isoler les polyorganosiloxanes comprenant les groupements fonctionnels **V**, par exemple au moyen d'une dévolatilisation standard, refroidissement et soutirage.

Ces polyorganosiloxanes peuvent constituer une excellente matière première pour préparer les polyorganosiloxanes **A** dépourvus de toxicité par contact cutané selon l'invention. Ainsi ces polyorganosiloxanes sont traités suivant l'invention de manière à fournir les polyorganosiloxanes **A** dépourvus de toxicité par contact cutané selon l'invention. Ces silicones aminées à groupements stériquement encombrés peuvent se présenter sous forme de solutions, de dispersions, de microémulsions ou d'émulsions.

Avantageusement, les polyorganosiloxanes **A** sont composés des motifs siloxyles représentés par la formule **(VIII)** suivante:

[(Z)(R¹⁵)₂SiO_{1/2}]_{2+w} [(R¹⁶)₂SiO_{2/2}]ₓ [(Z)(R¹⁷)SiO_{1/2}]_{y} [(Z)SiO_{3/2}]_{w} **(VIII)**

formule dans laquelle :
**(1)** les symboles Z, identiques ou différents, représentent R¹⁵ ou un groupement fonctionnel **V**;
**(2)** les symboles R¹⁵, R¹⁶ et R¹⁷, identiques ou différents, représentent chacun un radical alkyle en C₁-C₁₂, linéaire ou ramifié, éventuellement substitué, un radical cycloalkyle en C₅-C₁₀, éventuellement substitué, un radical aryle en C₆-C₁₈, éventuellement substitué, un radical aralkyle, éventuellement substitué ou un radical -OR¹⁸ où R¹⁸ représente un hydrogène, un radical alkyle, linéaire, ramifié ou cyclique, ayant de 1 à 15 atomes de carbone ou un groupement hydrocarboné comprenant une fonction époxyde et/ou polyéther ;
**(3)** les groupements fonctionnels **V** ont la même définition que ci-dessus (= groupement à fonction(s) pipéridinyle(s) stériquement encombrée(s)); et
**(4)**
   - le nombre de motifs siloxyles sans groupement fonctionnels **V** est compris entre 1 et 800,
   - le nombre de motifs siloxyles avec un groupement fonctionnel **V** est compris entre 1 et 50, et
   - 0 < w < 50 et 8 < y <798.

Selon un autre mode de réalisation préféré, les polyorganosiloxanes **A** de formule **(VIII)** sont linéaire (w=0) et les symboles R¹⁵, R¹⁶ et R¹⁷ de la formule **(VIII)** sont des groupements méthyles.

Selon un mode de réalisation particulièrement préféré, les polyorganosiloxanes **A** selon l'invention sont des composés de formule générale (**IX**) suivante : 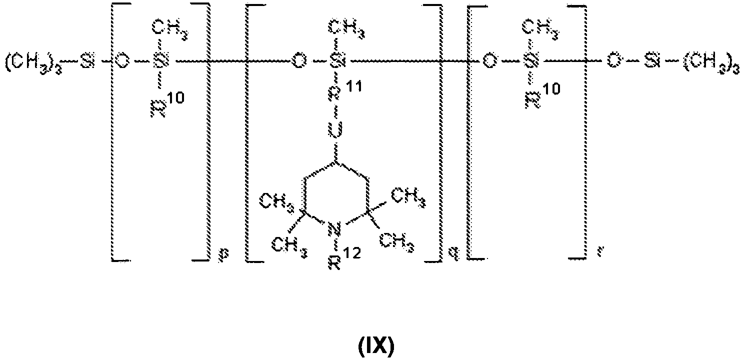 formule dans laquelle :
- les différents motifs sont répartis de manière statistique dans la chaîne ;
- p représente un nombre supérieur ou égal à 0 et de préférence compris entre 1 et 2000;
- r représente un nombre supérieur ou égal à 0 et de préférence compris entre 1 et 2000;
- q représente un nombre supérieur ou égal à 1, de préférence compris entre 1 et 50 ;
- R¹⁰ représente un radical alkyle, linéaire ou ramifié, ayant 1 à 18 atomes de carbone;
- le groupement U a la même signification que U', R¹¹ a la même signification que R'⁴ et R¹² a la même signification que R⁶ décrits ci-dessus.

Selon un mode préféré, la somme p+r est comprise entre 1 et 800.

Selon un autre mode de réalisation préféré, le groupement fonctionnel **V** est un radical propyloxytétraméthylpipéridine de formule (**X**) suivante:

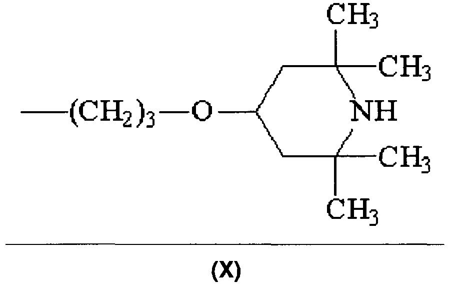

Les polyorganosiloxanes **A** dépourvus de toxicité par contact cutané dans lesquels 0,8 à 4% des atomes de silicium sont substitués par un groupement fonctionnel **V** selon l'invention sont particulièrement avantageux, notamment lorsqu'ils sont utilisés dans des compositions cosmétiques telles que décrites ci-dessous.

Selon un autre mode de réalisation particulièrement préféré, le groupement fonctionnel **V** est présent à raison de 2 à 4 % en poids par rapport, au poids total de l'huile silicone.

Un autre objet de l'invention concerne une composition cosmétique et/ou dermatologique dépourvue de toxicité par contact cutané caractérisée en ce qu'elle comprend dans un milieu cosmétiquement acceptable au moins un polyorganosiloxane **A** selon l'invention et tel que décrit ci-dessus.

Les compositions selon l'invention contiennent un milieu aqueux cosmétiquement acceptable. Elles présentent un pH pouvant aller de 3,5 à 11, de préférence de 5,5 à 11 et encore plus préférentiellement de 5,5 à 8,5.

Le "milieu cosmétiquement acceptable" (ou "vecteur cosmétiquement acceptable") des compositions selon l'invention est plus particulièrement constitué d'eau et éventuellement de solvants organiques cosmétiquement acceptables.

### "Milieu cosmétiquement acceptable" ou "vecteur cosmétiquement acceptable":

Tout milieu cosmétiquement acceptable permettant de formuler les polyorganosiloxanes **A** selon l'invention, et d'obtenir la forme de composition cosmétique désirée, pour l'utilisation visée, peut être utilisé. Différents milieu cosmétiquement acceptables pour différents types de formulations sont connus de l'homme du métier.

A titre d'exemples de vecteurs cosmétiquement acceptables, on peut citer les vecteurs aqueux (comprenant de l'eau), les vecteurs alcooliques (comprenant un alcool, par exemple de l'éthanol, l'isopropanol, l'éthylène glycol ou les polyéthylène glycol), le propylène glycol, les vecteurs hydro-alcooliques (comprenant un mélange d'eau et d'un alcool par exemple de l'éthanol, l'isopropanol, l'éthylène glycol ou les polyéthylène glycol). Certaines huiles, volatiles ou non, peuvent également être utilisées. On cite par exemple les silicones fluides, tels que le cyclopentasiloxane, par exemple le Mirasil CM5 commercialisé par Bluestar Silicones.

L'homme du métier sait choisir les vecteurs adaptés aux types de formulations souhaités, et aux utilisations visées. Par exemple des vecteurs aqueux sont généralement utilisés pour des shampoings ou gels-douche. Un vecteur propylène glycol peut être utilisé des compositions sous forme de crèmes. Un vecteur cyclométhicone peut être utilisé pour des compositions de maquillage, par exemple pour des fonds de teint.

### Tensioactifs:

La composition peut comprendre au moins un tensioactif. Il peut s'agir d'un mélange de différents tensioactifs. Il s'agit de préférence d'au moins un tensioactif anionique, seul ou en mélange. Le tensioactif peut en outre comprendre des tensioactifs anioniques, des tensioactifs amphotères (amphotères vrais ou zwitterioniques), des tensioactifs neutres et/ou des tensioactifs cationiques, seuls ou en mélange. Les compositions comprenant au moins un tensioactif amphotère et éventuellement un tensioactif anionique sont particulièrement avantageuses, notamment pour des raisons de douceur. La teneur totale en tensioactif dans la composition peut être comprise entre 5 et 30% en poids.

Pour des compositions destinées au traitement des cheveux, comme des shampoings, la teneur en tensioactif est avantageusement comprise entre 10 et 20% en poids. De telles compositions peuvent comprendre des sels, par exemple du chlorure de sodium ou d'ammonium, avantageusement en teneur inférieure à 3% en poids.

Pour des compositions destinées au traitement de la peau, comme des gel-douche, la teneur en tensioactif est avantageusement comprise entre 5 et 15% en poids. De telles compositions comprennent également de préférence au moins 2% en poids de sels, par exemple du chlorure de sodium ou d'ammonium.

La proportion en poids de tensioactif anionique par rapport à l'ensemble des tensioactifs est de préférence supérieure à 50%, de préférence supérieure à 70%.

Les tensioactifs anioniques peuvent être choisis parmi les tensioactifs suivants:
- les alkylesters sulfonates, par exemple de formule R-CH(SO₃M)-CH₂COOR' ou les alkylesters sulfates, par exemple de formule R-CH(OSO₃M)-CH₂COOR', où R représente un radical alkyle en C₈-C₂₀, de préférence en C₁₀-C₁₆, R' un radical alkyle en C₁-C₆, de préférence en C₁-C₃ et M un cation alcalino-terreux, par exemple sodium, ou le cation ammonium. On peut citer tout particulièrement les méthyl ester sulfonates dont le radical R est en C₁₄-C₁₆;
- les alkylbenzènesulfonates, plus particulièrement en C₉-C₂₀, les alkylsulfonates primaires ou secondaires, notamment en C₈-C₂₂, les alkylglycérol sulfonates ;
- les alkylsulfates par exemple de formule ROSO₃M, où R représente un radical alkyle ou hydroxyalkyle en C₁₀-C₂₄, de préférence en C₁₂-C₂₀ ; M un cation de même définition que ci-dessus ;
- les alkyléthersulfates par exemple de formule RO(OA)ₙSO₃M où R représente un radical alkyle ou hydroxyalkyle en C₁₀-C₂₄, de préférence en C₁₂-C₂₀ ; OA représentant un groupement éthoxylé et/ou propoxylé ; M représentant un cation de même définition que ci-dessus, n variant généralement de 1 à 4, comme par exemple le lauryléthersulfate avec n = 2 ;
- les alkylamides sulfates, par exemple de formule RCONHR'OSO₃M où R représente un radical alkyle en C₂-C₂₂, de préférence en C₆-C₂₀, R' un radical alkyle en C₂-C₃, M représentant un cation de même définition que ci-dessus, ainsi que leurs dérivés polyalcoxylés (éthoxylés et/ou propoxylés) (alkylamidoether sulfates
- les sels d'acides gras saturés ou insaturés, par exemple comme ceux en C₈-C₂₄, de préférence en C₁₄-C₂₀ et d'un cation alcalino-terreux, les N-acyl N-alkyltaurates, les alkyliséthionates, les alkylsuccinamates et alkylsulfo succinates, les monoesters ou diesters de sulfosuccinates, les N-acyl sarcosinates, les polyéthoxycarboxylates les mono et di esters phosphates, par exemple de formule suivante :
   (RO)ₓ-P(=O)(OM)ₓ ou R représente un radical alkyle, alkylaryle, arylalkyle, aryle,
   éventuellement polyalcoxylés, x et x' étant égaux à 1 ou 2, à la condition que la somme de x et
   x' soit égale à 3, M représentant un cation alcalino-terreux ;

Les tensioactifs non ioniques peuvent être choisis parmi les tensioactifs suivants:
- les alcools gras alcoxylés;
- les triglycérides alcoxylés;
- les acides gras alcoxylés;
- les esters de sorbitan alcoxylés;
- les amines grasses alcoxylées;
- les di(phényl-1 éthyl) phénols alcoxylés;
- les tri(phényl-1 éthyl) phénols alcoxylés;
- les alkyls phénols alcoxylés;
- les produits résultant de la condensation de l'oxyde d'éthylène avec un composé hydrophobe résultant de la condensation de l'oxyde de propylène avec le propylène glycol, tels les Pluronic commercialisés par BASF;
- les produits résultant de la condensation de l'oxyde d'éthylène le composé résultant de la condensation de l'oxyde de propylène avec l'éthylènediamine, tels les Tetronic commercialisés par BASF;
- les alkylpolyglycosides comme ceux décrits dans US 4565647; et
- les amides d'acides gras par exemple en C₈-C₂₀.

Les tensioactifs amphotères (amphotères vrais comprenant un groupe ioniques et un groupe potentiellement ionique de charge opposée, ou zwitterioniques comprenant simultanément deux charges opposées) peuvent être choisis parmi les tensioactifs suivants:
- les bétaïnes de manière générale, notamment carboxybétaïnes de par exemple la lauryl bétaïne (Mirataine BB de la société Rhodia) ou l'octylbétaïne; les amidoalkylbétaïnes, comme la cocamidopropyl bétaïne (CAPB) (Mirataine BDJ de la société Rhodia Chimie);
- les sulfo-bétaïnes ou sultaines comme la cocamidopropyl hydroxy sultaïne (Mirataine CBS de la société Rhodia);
- les alkylamphoacétates et alkylamphodiacétates, comme par exemple comprenant une chaîne coco ou lauryle (Miranol C2M, C32, L32 notamment, de la société Rhodia);
- les alkylamphopropionates ou les alkylamphodipropionates, (Miranol C2M SF); et
- les alkyl amphohydroxypropyl sultaïnes (Miranol CS).

Les tensioactifs cationiques peuvent être choisis parmi les sels d'amines grasses primaires, secondaires ou tertiaires, éventuellement polyethoxylées, les sels d'ammoniums quaternaires tels que les chlorures ou les bromures de tetraalkylammonium, d'alkylamidoalkylammonium, de trialkylbenzylammonium, de trialkylhydroxyalkylammonium, ou d'alkylpyridinium, les dérivés d'imidazoline, les oxydes d'amines à caractère cationique.

### Agent de stabilisation et/ou de conditionnement et/ou d'aide au conditionnement:

La composition cosmétique selon l'invention peut avantageusement comprendre au moins un agent de stabilisation et/ou de conditionnement (agents conditionneurs) et/ou d'aide au conditionnement. On parle aussi parfois d'agents de suspension. Par aide au conditionnement, on entend que la présence de l'agent améliore le conditionnement lié à d'autre composés, par exemple des huiles ou silicones. Les agents sont entendus comme des agents différents du polyorganosiloxane de formule (I). De tels agents sont connus de l'homme du métier. La composition selon l'invention peut comprendre plusieurs de ces agents (mélanges ou combinaisons), pour associer leurs effets et/ou créer des synergies. Par ailleurs, certains agents peuvent exercer plusieurs fonctions. C'est le cas par exemple des polysaccharides, et leurs dérivés cationiques, par exemple des dérivés cationiques de guars.

La proportion en poids de tels agents peut être typiquement de 0,1 % à 10% en poids, de préférence de 0,3% à 8% en poids, pour des polysaccharides ou d'autres agents.

A titre d'exemples d'agents de stabilisation (ou agents stabilisants), on peut citer:
- les polyacrylates réticulés, par exemple les polymères de type CARBOPOL ou CARBOMER commercialisés par BF Goodrich ou Noveon, ACRITAMER commercialisés par RITA ou TEGO CARBOMER commercialisés par Goldschmidt. Ces composés peuvent être typiquement présents en quantité de 0,1 à 3%, de préférence de 0,3 à 2%, en poids par rapport à la composition;
- les copolymères des acrylates/aminoacrylates/ itaconates PEG 20 alkyles C₁₀-C₃₀ commercialisés par National Starch sous le nom STRUCTURE PLUS. Ces composés peuvent être typiquement présents en quantité de 0,1 à 3%, de préférence de 0,3 à 2%, en poids par rapport à la composition; et
- les solides insolubles formant un réseau dans la composition. Il peut s'agir de mono et/ou diesters d'acides gras d'éthylène glycol, les acides gras étant de préférence en C₁₆-C₁₈. Il peut en particulier s'agir d'éthylène glycol distéarate (EGDS), par exemple commercialisé par Rhodia en concentré avec d'autres ingrédients sous le nom MIRASHEEN. Ce composé peut être typiquement présent en quantité de 3 à 10%, de préférence de 5 à 8% en poids par rapport à la composition.

On peut citer également des agents viscosants, gelifiants ou texturants comme les copolymères acryliques anioniques de type ACULYNE commercialisés par ISP ou Rohm & Haas), les polysaccharides et leurs dérivés non cationiques tels que les dérivés de la cellulose comme l'hydroxypropylcellulose, la carboxyméthylcellulose, les dérivés non-ioniques de guars comme l'hydroxypropyl guar (par exemple les Jaguar HP commercialisé par Rhodia), la caroube, la gomme de tara ou de cassia, la gomme xanthane (par exemple les Rhodicare commercialisés par Rhodia), les succinoglycanes (par exemple le Rheozan commercialisé par Rhodia), les alginates, les carraghénannes, les dérivés de la chitine ou tout autre polysaccharide à fonction texturante. Ces polysaccharides et leurs dérivés peuvent être incorporés seuls ou en combinaison synergique à d'autres polysaccharides. Ces composés peuvent être typiquement présents en quantité de 0,1 à 3%, de préférence de 0,3 à 1%, en poids par rapport à la composition.

A titre d'exemples d'agents de stabilisation et/ou d'agents de conditionnement et/ou d'aide ou conditionnement, on peut citer:
- les polymères cationiques dérivés de polysaccharides, par exemple les dérivés cationiques de celluloses, les dérivés cationiques d'amidons, les dérivés cationiques de guars, les dérivés cationique de caroube;
- les polymères cationiques synthétiques; et
- les mélanges ou combinaisons de ces agents.

Les polymères cationiques, synthétiques ou non, pouvant assurer une fonction d'agent de conditionnement, sont notamment des polymères de type polyquaternium, comme par exemple les polyquaternium-1, polyquaternium-2, polyquaternium-4, polyquaternium-5, polyquaternium-6 (également connu comme Merquat 1000 disponible auprès de Nalco), polyquaternium-7 (également connu comme Merquat 5500 disponible auprès de Nalco), polyquaternium-8, polyquaternium-9, polyquaternium10 (également connu comme Polymer JR 400, commercialisé par Amercol), polyquaternium-11, polyquaternium-12, polyquaternium-13, polyquaternium-14, polyquaternium-15, polyquaternium-16, polyquaternium-17, polyquaternium-18, polyquaternium-19, polyquaternium-20, polyquaternium-22 (également connu comme Merquat 280, 281, 298 disponibles auprès de Nalco), polyquaternium-24, polyquaternium-27, polyquaternium-28, polyquaternium-29 (également connu comme Kytamer KCO disponible auprès de Amerchol), polyquaternium-30, polyquaternium-31, polyquaternium-32, polyquaternium-33, polyquaternium-34, polyquaternium-35, polyquaternium-36, polyquaternium-37, polyquaternium-39 (également connu comme Merquat 3300, 3331 disponibles auprès de Nalco), polyquaternium-44, polyquaternium-27 (également connu comme Merquat 2001 disponible auprès de Nalco) et polyquaternium-55.

Les dérivés cationiques des guars peuvent avoir une fonction d'agent de stabilisation des formulations, d'agent de conditionnement et/ou d'agent d'aide au conditionnement. A titre d'exemples on peut citer:
- le guar hydroxypropyl trimonium chlorure, (Jaguar C 13S, Jaguar C14S, Jaguar C17, Jaguar Excel, Jaguar C 2000, commercialisés par RHODIA);
- l'hydroxypropyl guar hydroxypropyl trimonium chlorure (Jaguar C162, commercialisés par RHODIA);
- l'éther de poly(oxyéthanediyl-1,2)hydroxy-2 chlorure de triméthylammonium-3 propyl cellulose ou polyquaternium-10.

### Autre polyorganosiloxane:

La composition selon l'invention peut comprendre un polyorganosiloxane différent des polyorganosiloxanes **A**. Cet autre polyorganosiloxane peut être par exemple présent sous forme d'une émulsion, définissant dans le vecteur cosmétiquement acceptable une deuxième famille de gouttelettes (on parle souvent de co-émulsions). Il peut également être en mélange avec les polyorganosiloxanes **A** sous forme d'une émulsion de gouttelettes du mélange (on peut parler d'émulsions de mélanges), ou sous forme de gouttelettes dispersées dans les polyorganosiloxanes **A** (on peut parler d'émulsions multiples).

Le polyorganosiloxane différent des polyorganosiloxanes **A** peut être un polyorganosiloxane comprenant des groupes polaires, ou un polyorganosiloxane non polaire.

A titre d'exemples de polyorganosiloxanes comprenant des groupes polaires, on peut citer: les dimethiconols, amodimeticones, trimethylsilyl amodimethicone, dimethicone copolyols, ternary copolyols, Silatrizole, dimethicone copolyol amine, silicone quaternium (CTFA silicone quaternium 1 à 10).

A titre d'exemples de polyorganosiloxanes non polaires, on peut citer les polydimethylorganosiloxanes (PDMS ou diméthicone), les silicones présentant des groupes phényl, les silsesquioxane (structure «T») & silicates (structure «Q»), les silicones réticulées, les copolymères comprenant des groupes silicones, les résines silicones, les cires silicones, les siloxanes alkyle methyle volatiles.

De même que pour les polyorganosiloxanes **A**, les émulsions de polyorganosiloxane différent des polyorganosiloxanes **A** peuvent être préparées par émulsification in situ ou par émulsification préalable, et présenter des tailles de gouttelettes inférieures à 0,15 µm, ou comprises entre 0,15µm et 2 µm, ou supérieures ou égales à 2µm. On se réfère au passage consacré aux émulsions ci-après.

### Filtres UV

La composition selon l'invention peut comprendre des agents de filtres UV. Il peut s'agir de d'agents organiques, ou minéraux. Il peut par exemple s'agir d'agents minéraux tels que des dispersions de particules à base de dioxyde de titane, d'oxyde de zinc, ou d'oxyde de cérium, de préférence sous forme nanoparticulaire, le cas échéant recouvertes d'une couche à base d'oxyde ou hydroxyde de silice ou d'aluminium, par exemple la dispersion commercialisée sous la dénomination Mirasun^{®} TiW60 par Rhodia. Il peut également s'agir de molécules organiques. De telles molécules sont connues de l'homme du métier. A titre d'exemples de molécules organiques, on cites les composés suivants: Eusolex OCR ou Eusolex 6300 (Merck); Parsol 1789, Parsol HS, ou Parsol MCX (Givaudan Roure); Mexoril SX (Chimex); Escalol 567, Escalol 587, ou Escalol 507 (ISP/Van Dyk); Uvinul MS-40, Uvinul T-150, ou Spectrasorb UV-24 (BASF); Neo Heliopan MA ou Neo Heliopan Type E 1000 (Haarmann & Reimer); Tinosorb M (Ciba), Homomenthyl salicylate.

### Autres ingrédients:

A titre d'autres ingrédients pouvant être compris dans la composition, on peut citer des agents de coloration, teintures ou colorants, des fragrances, des parfums, des agents masquant les fragrances, des polymères, des agents tampons, des complexants, des capsules complexantes, des sels solubles, par exemple des sels de métaux, d'alcalins, d'alcalino-terreux, ou d'ammoniums, par exemple NaCl ou NaSO₄ ou NH₄Cl, des acides de Lewis, des épaississants particulaires, des épaississants polymériques, des cires épaississantes, des huiles, des agents émollients, des humectants, des agents hydratants, des agents nacrants, des opacifiants, des agents dispersants, des agents favorisant la suspension de particules, des agent anti-microbiens, des conservateurs, des protéïnes, des extraits végétaux, des oxydants, des agents modifiant la viscosité, des agents de gélification, des chélatants, des réducteurs.

La composition peut en outre comprendre une grande variété d'agents actifs, hydrophiles ou non. Il peut s'agir d'agents antifongiques, anti-bactériens, par exemple le triclosan, d'agents antipelliculaires, par exemple du zinc-pyrithione, d'agents anti-vieillissement, d'agents anti-cellulite.

A titre d'exemples de matières actives utilisables dans le domaine de la cosmétique on peut citer les vitamines, comme la vitamine A et ses dérivés notamment ses esters comme l'acétate, le palmitate, le propionate, la vitamine B2, l'acide pantothénique, la vitamine D et la vitamine E ; les mono-, di- et triglycérides ; les bactéricides ; les agents absorbeurs d'UV, comme les dérivés aminobenzoate de type PABA et PARA, les salicylates, les cinnamates, les anthranilates, les dibenzoylméthanes, les dérivés du camphre et leurs mélanges.

Les agents anti-vieillissement peuvent de même être utilisés. A titre d'exemples de tels agents on peut citer notamment les rétinoïdes, les vitamines liposolubles, les dérivés de la vitamine C comme les esters notamment l'acétate, le propionate, le palmitate ; les céramides, les pseudo-céramides, les phospholipides, les acides gras, les alcools gras, le cholestérol, les stérols et leurs mélanges. Comme acides gras et alcools préférés, on peut plus particulièrement citer ceux possédant des chaînes alkyles, linéaires ou ramifiées contenant de 12 à 20 atomes de carbone. Il peut notamment s'agir d'acide linoléique.

On peut de même mettre en oeuvre des agents anti-cellulite, tels que notamment l'isobutylméthylxanthine et la théophyline ; ainsi que des agents anti-acné, comme par exemple le résorcinol, l'acétate de résorcinol, le peroxyde benzoyle et de nombreux composés naturels.

Les arômes, parfums, huiles essentielles, essences, peuvent aussi être utilisés en tant que matière active. A titre d'exemple, on peut citer les huiles et/ou essences de menthe, de menthe verte, de menthe poivrée, de menthol, de vanille, de cannelle, de laurier, d'anis, d'eucalyptus, de thym, de sauge, de feuille de cèdre, de noix de muscade, de citrus (citron, citron vert, pamplemousse, orange), de fruits (pomme, poire, pêche, cerise, prune, fraise, framboise, abricot, ananas, raisin, etc.), seules ou en mélanges. On peut aussi mettre en oeuvre des composés comme le benzaldéhyde, l'acétate d'isoamyle, le butyrate d'éthyle, etc.

Les agents anti-microbiens peuvent être choisis parmi le thymol, le menthol, le triclosan, le 4-hexylrésorcinol, le phénol, l'eucalyptol, l'acide benzoïque, le peroxyde benzoïque, le parabène de butyle, et leurs mélanges.

Les compositions cosmétiques peuvent également contenir des polymères présentant des propriétés filmogènes pouvant être utilisés pour apporter une fonction fixante. Ces polymères sont généralement présents à des concentrations comprises entre 0,01 et 10 %, préférentiellement entre 0,5 et 5 %. Ils sont préférentiellement du type polyvinylpyrrolidone, copolymères de polyvinylpyrrolidone et de méthyl méthacrylate, copolymère de polyvinylpyrrolidone et d'acétate de vinyle, copolymères polytéréphtalate d'éthylène glycol/polyéthylène glycol, polymères copolyesters téréphtaliques sulfonés.

On peut également incorporer aux compositions cosmétiques des agents hydratants. A titre illustratif de ces derniers, on peut notamment citer le glycérol, le propylène glycol, l'urée, le collagène, la gélatine, et des émollients qui sont généralement choisis parmi les alkylmonoglycérides, les alkyldiglycérides, les triglycérides comme les huiles extraites des plantes et des végétaux ou leurs dérivés hydrogénés, les huiles minérales ou les huiles paraffiniques, les diols, les esters gras, les silicones (voir plus haut).

Des agents conservateurs comme les esters de l'acide p-hydroxybenzoïque, le benzoate de sodium, ou tout agent chimique évitant la prolifération bactérienne ou des moisissures et utilisé traditionnellement dans les compositions cosmétiques sont généralement introduits dans ces compositions à hauteur de 0,01 à 3 % en poids. Des agents conservateurs sont par exemple commercialisés sous les noms Glydant, Germaben, Kathon.

### Forme physico-chimique de la composition cosmétique:

La composition cosmétique comprend un vecteur cosmétiquement acceptable et les polyorganosiloxanes **A** selon l'invention. Les polyorganosiloxanes dispersés dans le vecteur cosmétiquement acceptable, ou dans un mélange d'ingrédients comprenant le vecteur cosmétiquement acceptable. La dispersion peut par exemple être:
- une solution des polyorganosiloxanes **A** selon l'invention dans le vecteur cosmétiquement acceptable ou dans un mélange comprenant le vecteur cosmétiquement acceptable;
- une émulsion stable de gouttelettes comprenant les polyorganosiloxanes **A** selon l'invention dans le vecteur cosmétiquement acceptable, ou
- une association de phases séparées en au moins une strate comprenant le vecteur cosmétiquement acceptable, et un strate comprenant les polyorganosiloxanes **A** selon l'invention, pouvant former une dispersion de gouttelettes comprenant les polyorganosiloxanes **A** selon l'invention dans le vecteur cosmétiquement acceptable après agitation par l'utilisateur.

Bien entendu, le vecteur cosmétiquement acceptable peut comprendre d'autres ingrédients que les polyorganosiloxanes **A** selon l'invention, ces autres ingrédients pouvant être présents en solution ou en dispersion, par exemple sous forme d'une suspension de particules solides, ou d'une émulsion définissant une famille de gouttelettes.

De même, les polyorganosiloxanes **A** selon l'invention peuvent définir une phase dans laquelle sont dispersés un ou plusieurs autres ingrédients.

On détaille ci-dessous quelques caractéristiques de compositions cosmétiques selon l'invention sous forme d'émulsions.

### Emulsions:

Les compositions cosmétiques selon l'invention peuvent être sous forme d'émulsions de gouttelettes comprenant les polyorganosiloxanes **A** selon l'invention dispersées dans le vecteur cosmétiquement acceptable, de préférence dans un vecteur aqueux.

Les gouttelettes de l'émulsion peuvent être de taille plus ou moins importante. On peut ainsi se référer à des microémulsions, à des mini-émulsions, ou à des macro-émulsions. Dans la présente demande, le terme «émulsion» couvre notamment tous ces types d'émulsions. Sans vouloir être lié à une quelconque théorie, on précise que les microémulsions sont généralement des systèmes thermodynamiquement stable, comprenant généralement d'importantes quantités d'agents d'émulsification. Les autres émulsions sont généralement des systèmes en état non-thermodynamiquement stable, conservant pendant un certain temps, en état métastable l'énergie mécanique fournie lors de l'émulsification. Ces systèmes comprennent généralement des quantités moindres d'agents d'émulsification.

Les compositions sous forme d'émulsions peuvent être obtenues par mélange du vecteur, de préférence aqueux, les polyorganosiloxanes **A** selon l'invention, et en général d'un agent d'émulsification, puis émulsification. On peut parler d'émulsification in situ.

Les compositions sous forme d'émulsion peuvent également être obtenues par mélange du vecteur, de préférence aqueux, avec une émulsion préalablement préparée de gouttelettes comprenant les polyorganosiloxanes **A** selon l'invention dans une phase externe, de préférence miscible avec le vecteur cosmétiquement acceptable, de préférence de même nature que ledit vecteur, de préférence un vecteur aqueux. On peut préférer ce mode de réalisation car il est simple à mettre en oeuvre. En outre ce mode de réalisation est particulièrement adapté pour la mise en oeuvre de compositions cosmétiques dans lesquelles le polyorganosiloxane de formule (I) est sous forme de microémulsion. On peut parler d'émulsification préalable.

Selon un mode particulier de réalisation, l'émulsion est une microémulsion, dont la taille des gouttelettes est inférieure à 0,15 µm. Dans ce mode de réalisation, la composition comprend de préférence de préférence en proportion supérieure à 10% en poids, de préférence au moins 15% en poids d'agent d'émulsification par rapport au poids des polyorganosiloxanes **A**.

La taille des gouttelettes de microémulsion peut être mesurée sur une émulsion préparée préalablement à son introduction dans la composition cosmétique, par diffusion de Lumière Dynamique (DQEL), par exemple comme décrit ci-après. L'appareillage utilisé est par exemple constitué d'un laser Spectra-Physics 2020, d'un corrélateur Brookhaven 2030 et de l'informatique associée. L'échantillon étant concentré, il est dilué dans l'eau désionisée et filtré à 0,22 µm, pour être en final à 2% en poids. Le diamètre obtenu est un diamètre apparent. Les mesures sont faites à 90° et 135° d'angle. Pour les mesures de taille, outre l'analyse classique par les cumulants, trois exploitations de la fonction d'autocorrélation sont utilisées (l'échantillonnage exponentiel ou EXPSAM décrit par le Pr. Pike, la méthode « Non Negatively Constrained Least Squares » ou NNLS et la méthode CONTIN décrite par le Pr. Provencher), qui donnent chacune une répartition de taille pondérée par l'intensité diffusée, et non pas par la masse ou le nombre. Il est tenu compte de l'indice de réfraction et de la viscosité de l'eau.

Selon un mode avantageux, la microémulsion est transparente. La microémulsion peut par exemple présenter une transmittance d'au moins 90%, de préférence d'au moins 95%, à une longueur d'onde de 600 nm, mesurée par exemple à l'aide d'un spectromètre Lambda 40 UV-Vis, à une concentration à 0.5% en poids dans l'eau. Dans ce cadre, la composition cosmétique peut avantageusement être transparente. Elle peut par exemple présenter une transmittance d'au moins 90%, de préférence d'au moins 95%, à une longueur d'onde de 600 nm, mesurée par exemple à l'aide d'un spectromètre Lambda 40 UV-Vis.

Selon un autre mode particulier de réalisation, l'émulsion est une émulsion dont la taille moyenne des gouttelettes est supérieure ou égale à 0,15 µm, par exemple supérieure à 0,5 µm, ou à 1 µm, ou à 2 µm, ou à 10 µm, ou à 20 µm, et de préférence inférieure à 100 µm. La taille des gouttelettes peut être mesurée sur une émulsion préparée préalablement à son introduction dans la composition cosmétique, ou directement sur la composition cosmétique diluée dans de l'eau, par microscopie optique et/ou granulomètrie laser (Horiba LA-910 laser scattering analyzer). Dans ce mode de réalisation, la composition comprend de préférence en proportion inférieure à 10% en poids d'agent d'émulsification, par rapport au poids des polyorganosiloxanes **A** selon l'invention.

Comme mentionné ci-dessus les gouttelettes de l'émulsion peuvent comprendre d'autres ingrédients que les polyorganosiloxanes **A** selon l'invention. Ainsi, les polyorganosiloxanes **A** selon l'invention peuvent être mélangés à un ingrédient miscible, par exemple une huile, de préférence une huile silicone, le mélange formant l'émulsion. Les gouttelettes de polyorganosiloxanes **A** selon l'invention peuvent également comprendre une émulsion de gouttelettes de plus petite taille d'une phase non miscible (phase interne). L'émulsion est alors une émulsion multiple comprenant une phase interne dispersée dans une phase intermédiaire comprenant les polyorganosiloxanes **A** selon l'invention, elle-même dispersée dans le vecteur. Les ingrédients pouvant être compris dans la phase interne peuvent par exemple être des ingrédients actifs procurant un effet positif sur la peaux et/ou les cheveux. Il peut s'agir également d'agents favorisant le dépôt des polyorganosiloxanes **A** selon l'invention, ou d'autres ingrédients, sur la peau et/ou les cheveux.

### Agents d'émulsification:

Les agents d'émulsification sont des agents pouvant permettre d'obtenir une émulsion des polyorganosiloxanes **A** selon l'invention dans le vecteur, de préférence de l'eau. Il peut par exemple s'agir:
- d'un tensioactif non ionique,
- d'un polymère amphiphile non ionique, éventuellement associé à un ou plusieurs tensioactifs anioniques et/ou polymères amphiphiles anioniques,
- d'un tensioactif particulaire éventuellement associé à un co-tensioactif, ou
- d'un colloïde protecteur.

### Tensioactif particulaire:

Selon un mode particulier, l'agent d'émulsification est un tensioactif particulaire éventuellement associé à un co-tensioactif.

Le tensioactif particulaire est de préférence choisi parmi les composés particulaires solides, dont l'angle de contact est proche de 0°, associés à au moins un co-stabilisants choisi parmi les tensioactifs non-ioniques, anioniques, cationiques ou zwitterioniques.

Le tensioactif particulaire est par exemple une silice de précipitation, une silice colloïdale, un silicoaluminate, de l'oxyde de zinc, de l'oxyde de titane, ou un mélange de ces composés, ces composés comprenant le cas échéant un traitement de surface.

### Colloïde protecteur:

Selon un autre mode particulier l'agent d'émulsification est un colloïde protecteur. Il peut par exemple s'agir d'un alcool polyvinylique, le cas échéant partiellement hydrolysé.

La teneur en colloïde protecteur est avantageusement de 3 à 30 % en poids sec par rapport à l'émulsion interne, de préférence de 5 à 25%.

### Tensioactif non ionique

Selon un autre mode particulier l'agent d'émulsification comprend un tensioactif non ionique. Il s'agit de préférence d'un tensioactif non ionique polyalcoxylé, par exemple choisi parmi le groupe constitué par:
- les alcools gras alcoxylés;
- les triglycérides alcoxylés;
- les acides gras alcoxylés;
- les esters de sorbitan alcoxylés;
- les amines grasses alcoxylées;
- les di(phényl-1 éthyl) phénols alcoxylés;
- les tri(phényl-1 éthyl) phénols alcoxylés; et
- les alkyls phénols alcoxylés,
où le nombre de motifs alcoxy, plus particulièrement oxyéthyléne et/ou oxypropyléne, est tel que la valeur de HLB est supérieure ou égale à 10.

### Polymère amphiphite non-ionique:

Selon un autre mode particulier l'agent d'émulsification externe comprend un polymère amphiphile non ionique. Ce polymère peut être associé à un ou plusieurs tensioactifs anioniques et/ou polymères amphiphiles anioniques.

A titre de polymères amphiphiles non ionique on cite les copolymères triblocs (polyéthylène glycol) - (polypropylène glycol) - (polyéthylène glycol).

Pour ce qui a trait aux polymères amphiphiles non ioniques ou anioniques, on peut mettre en oeuvre un polymère comprenant au moins deux blocs, l'un d'eux étant hydrophile, l'autre hydrophobe. On peut mettre en oeuvre un copolymère peignes.

Lesdits polymères amphiphiles peuvent de manière avantageuse, être obtenus par polymérisation radicalaire dite vivante ou contrôlée. A titre d'exemples non limitatifs de procédés de polymérisation dite vivante ou contrôlée, on peut notamment se référer aux demandes WO 98/58974, WO 00/75207 et WO 01/42312 (xanthate), WO 98/01478 (dithioesters), WO 99/03894 (nitroxydes) ; WO 99/31144 (dithiocarbamates), WO 02/26836 (dithiocarbazates) ; WO 02/10223 (dithiophosphoroesters), WO 96/30421 (polymérisation radicalaire par transfert d'atome - ATRP). Les polymères amphiphiles peuvent aussi être obtenus par polymérisation anionique. Ils peuvent de même être préparés en mettant en jeu des polymérisations par ouverture de cycle (notamment anionique), ou par modification chimique du polymère.

Plus particulièrement, en ce qui concerne le polymère amphiphile non ionique, de préférence polyoxyalkyléné, présent de la phase aqueuse externe, il peut être choisi parmi les polymères miscibles au moins en partie dans la phase aqueuse externe et de préférence parmi les copolymères triblocs polyéthylène glycol-polypropylène glycol-polyéthylène glycol. Il est précisé que des polymères de type alcool polyvinylique ou triblocs polyacide acrylique/polyacrylate de butyle/polyacide acrylique peuvent être utilisés à cette fin.

### Type de formulation de la composition et utilisations:

La composition selon l'invention peut être formulée sous différentes formes, dépendant de l'aspect que l'on souhaite lui conférer, des propriétés sensorielles (viscosité, toucher, permanence ...) que l'on souhaite lui conférer, et bien entendu de l'utilisation que l'on souhaite en faire. Les différents types de formulations et les différentes utilisations sont modulés par la nature et la quantité des ingrédients présents dans la composition, et sont connus de l'homme du métier.

Ainsi la composition peut être formulée sous forme de gels, de fluides plus ou moins visqueux, de laits, de crèmes, d'huiles, de sprays, de mousses, de gels en bâtonnets (sticks), de pâtes, de lotions, de concentrés de teinture etc...

Les compositions peuvent notamment être choisies parmi les compositions listées dans le tableau I ci-dessous, avec des formes physico-chimiques des polyorganosiloxanes **A**, des types de formulation et des utilisations également listées dans le tableau (I) ci-dessous. Pour ces compositions, formes physico-chimiques, type de formulations et utilisations, on peut se reporter à des parties plus détaillées de la présente demande.

**Tableau I**

| Composition | Forme physico-chimique silicones A selon l'invention | Type de formulation | Utilisation |
|---|---|---|---|
| Shampoings | Emulsion | Fluide | Nettoyage et/ou soin des cheveux et/ou coloration temporaire et/ou fixation de la coloration, avec rinçage |
| Après-shampoing | Emulsion | Fluide | Soin des cheveux, et/ou démêlage et/ou aide au coiffage et/ou coloration temporaire et/ou fixation de la coloration et/ou conditionnement et/ou conditionnement après coloration, avec ou sans rinçage |
| Gel-douche | Emulsion | Fluide ou Gel | Nettoyage et/ou soin de la peau |
| Masque capillaire | Emulsion | Fluide très visqueux | Soin des cheveux |
| Crème solaire | Emulsion | Crème | Protection de la peau contre les UV |
| Lait Solaire | Emulsion | Lait | Protection de la peau contre les UV |
| Huile Solaire | Emulsion inverse ou solution | Huile | Protection de la peau contre les UV |
| Spray solaire | Emulsion | Fluide | Protection de la peau contre les UV |
| Crème de soin | Emulsion | Crème | Soin de la peau |
| Démaquillant | Emulsion | Crème ou fluide ou gel | Soin et/ou nettoyage de la peau et/ou des cils |
| Maquillage | Emulsion, ou suspension inverse ou solution | Crème, fluide, mascara, poudre, Gels, Bâtonnets | Coloration de la peau ou des cils |
| Déodorant | Emulsion ou Emulsion inverse | Aérosol, Gel, Bâtonnets, substance pouvant être appliquée à l'aide d'un applicateur à boule | Réduction des effets de la transpiration, appliqué sur la peau |
| Mousse à raser | Emulsion | Liquide très fluide ou gel formant une mousse après propulsion aérosol | Préparation au rasage |
| | | | |
| Spray Coiffant ou fixant | Emulsion | Fluide | Mise en forme des cheveux |
| Gel Coiffant ou fixant | Emulsion | Gel | Mise en forme des cheveux |
| Mousse coiffante ou fixante | Emulsion | Liquide très fluide ou gel formant une mousse après propulsion aérosol | Mise en forme des cheveux |
| Composition tinctoriale | Emulsion | Gel ou liquide visqueux | Teinture permanente ou semi-permanente |

Parmi les utilisations des compositions, on peut mentionner les utilisations dans lesquelles la composition est destinée à être rincée et les utilisations dans lesquelles la composition est destinée à ne pas être rincée.

### Compositions destinées à être rincées («rinse-off»):

Selon des modes de réalisation intéressants, la composition est une composition pour le soin de la peau et/ou des cheveux, de préférence pour de nettoyage et/ou le traitement et la peau et/ou des cheveux, ladite composition étant sous forme d'un fluide. Il s'agit avantageusement d'un gel douche, d'un shampoing, d'un après-shampoing destiné à être rincé, d'un masque cutané ou capillaire, destiné à être rincé après utilisation.

Pour les gels-douche et shampoings, la composition peut avantageusement comprendre:
- au moins un tensioactif anionique et/ou amphotère, seul ou en mélange,
- éventuellement, au moins un agent de stabilisation et/ou de conditionnement et/ou d'aide au conditionnement, ou un mélange de tels agents,
- éventuellement un autre polyorganosiloxane,
- des mélanges de ces ingrédients.

De tels ingrédients ont été décrits plus haut.

Pour les après-shampoings destinés à être rincés, la composition peut avantageusement être une formulation assez visqueuse, par exemple une crème, sous forme d'une émulsion comprenant une phase aqueuse dans laquelle sont dispersées une phase émulsionnée huileuse texturante, et des gouttelettes émulsionnées des polyorganosiloxanes **A** selon l'invention. La phase aqueuse comprend avantageusement un agent de conditionnement, par exemple un polymère cationique. De tels polymères ont été décrits plus hauts. La phase aqueuse peut également avantageusement comprendre un tensioactif cationique. De tels tensioactifs ont été décrits plus haut. Il peut s'agir par exemple de chlorure de stearyl benzyl dimethyl ammonium, de chlorure de cetyl trimethyl ammonium (chlorure de cetrimonium), de chlorure de distearyldimethyl ammonium or stearamidopropyldimethylamine, par exemple en quantité 0.3 à 2% en poids.

### Compositions destinées à ne pas être rincées («leave-on»):

Selon des modes de réalisation intéressants, la composition est une composition pour le soin de la peau et/ou des cheveux, sous forme d'un fluide ou sous une autre forme, de préférence pour le traitement et/ou la protection et/ou la modification de l'aspect de la peau et/ou des cheveux, destinée à être laissée sur la peau et/ou les cheveux après application.

Il peut par exemple s'agir d'un après-shampoing destiné à ne pas être rincé, d'un lait démêlant, d'une eau démêlante, d'une eau de lissage, d'un revêtement de cuticule («cuticle coat»), d'un produit de soin capillaire coiffant, d'un produit de soin capillaire coiffant et recoiffant, d'un produit de protection solaire (crème solaire, lait solaire, huile solaire), d'une crème de soin, d'un démaquillant, d'un maquillage, de lingettes démaquillantes ou hydratantes, de mousses à raser, de mousses coiffantes ou fixantes, de gels coiffants ou fixants.

Les gels-douche, shampoing ou après-shampoings, destinés à être rincés ou non, comprenant les polyorganosiloxanes **A** selon l'invention, peuvent ainsi présenter des améliorations en matière de:
- fixation des colorations opérées avant ou pendant l'application de la composition,
- conditionnement des cheveux, particulièrement sur cheveux abîmé et/ou sur les pointes,
- conditionnement de la peau,
- modulation du conditionnement des cheveux et/ou de la peau (conditionnement doux ou important)
- modulation du conditionnement des cheveux et/ou de la peau en fonction du taux d'azote présent dans le polyorganosiloxane,
- effets cosmétiques tels que la douceur, la souplesse, le démêlage, la brillance, l'aptitude au coiffage sur cheveux secs ou mouillés,
- faible jaunissement,
- réparation d'endommagement lié au soleil, à des décolorations liées au soleil ou à d'autres conditions extérieures, ou à une usure,
- préservation et/ou faible dégradation de composés compris dans la composition,
- longue tenue d'un actif délivré sur la peau et/ou les cheveux.

les polyorganosiloxanes **A** selon l'invention peuvent en particulier être utilisé dans des compositions destinées aux traitement de cheveux avant été exposés ou étant exposés à compositions de teintures comprenant un agent d'oxydation, typiquement des compositions de teinture durable comprenant une base d'oxydation ou des compositions pour la décoloration ou l'éclaircissement des cheveux, comprenant un agent d'oxydation. A ce titre, il peut s'agir d'un shampoing, d'un après shampoing, ou d'une composition de traitement ou conditionnement des cheveux après teinture.

La composition selon l'invention peut être une composition pour la teinture des cheveux. De telles compositions sont connues de l'homme du métier. On précise que les compositions pour la teinture des cheveux peuvent être constituées de plusieurs produits pour la teinture des cheveux, destinés à être mélangés par l'utilisateur. Dans la présente demande, sauf mention contraire ou précision particulière, le terme «composition pour la teinture de cheveux» couvre aussi bien une composition complète, ou un produit destiné à être mélangé à un autre par l'utilisateur. Dans la présente demande, le terme «teinture des cheveux», couvre toute modification de la couleur des cheveux, qu'il s'agisse d'une coloration proprement dite, d'une décoloration, ou d'une combinaison de décoloration et d'une coloration.

La composition pour la teinture des cheveux peut comprendre une base d'oxydation (précurseurs de colorants d'oxydation). Elle peut comprendre un agent d'oxydation. Elle peut comprendre un agent coupleur (modificateur de coloration). Elle peut comprendre un agent de coloration directe (colorants directs). La composition comprend un vecteur cosmétiquement acceptable. La composition peut comprendre également des adjuvants.

Selon un mode de réalisation, il s'agit d'une composition pour une teinture durable comprenant une base d'oxydation, un agent d'oxydation, et éventuellement un agent coupleur, de préférence sous forme de deux produits à associer, un produit comprenant la base d'oxydation et un produit comprenant l'agent d'oxydation.

Selon un mode de réalisation, il s'agit d'une composition pour une teinture temporaire ou durable comprenant un agent de coloration directe, et éventuellement un agent d'oxydation.

Selon un mode de réalisation, il s'agit d'une composition pour la décoloration ou l'éclaircissement des cheveux, comprenant un agent d'oxydation.

A titre d'agents de coloration directe, on peut citer les colorants nitrés benzéniques neutres, acides ou cationiques, les colorants directs azoïques neutres, acides ou cationiques, les colorants directs quinoniques et en particulier anthraquinoniques neutres acides ou cationiques, les colorants directs aziniques, les colorants directs méthiniques, les colorants directs tétraazapentaméthiniques, les colorants directs triarylméthaniques, les colorants directs indoaminiques et les colorants directs naturels.

A titre d'agents d'oxydation, on peut citer le peroxyde d'hydrogène, le peroxyde d'urée, les bromates de métaux alcalins, les persels tels que les perborates et les persulfates, les peracides et les enzymes, notamment les peroxydases, les oxydo-réductases à 2 électrons, et les oxygénases à 4 électrons.

A titre d'agents coupleurs, on peut citer les méthaphénylènediamines, les méta-aminophénols, les métadiphénols, les coupleurs naphtaléniques et les coupleurs hétérocycliques.

A titre de vecteurs cosmétiquement acceptables, préférés dans les compositions de teinture, on peut citer l'eau et/ou ses mélanges avec des solvants, par exemple l'éthanol, l'isopropanol, les polyols et éthers de polyols comme le 2-butoxyéthanol, le propylène glycol, les alcools aromatiques comme l'alcool benzylique ou le phénoxyéthanol.

Les adjuvants peuvent être des tensioactifs anioniques, non-ioniques, cationiques ou zwitterioniques ou amphotères, des polymères anioniques, neutres ou cationiques, des agents épaississants minéraux ou organiques, des agents anti-oxydants, des agents de pénétration, des agents séquestrants, des parfums, des tampons, des agents dispersants, des agents de conditionnement, des agents filmogènes, des céramides, des agents conservateurs, des opacifiants. Bien entendu les ingrédients mentionnés plus haut peuvent être utilisés comme adjuvants dans les compositions pour la teinture teinture.

Les compositions pour la teinture des cheveux comprenant les polyorganosiloxanes A selon l'invention peuvent ainsi :
- prévenir un éclaircissement de la coloration dans le temps (fading),
- favoriser une tenue de la coloration dans le temps,
- diminuer l'extraction de la couleur, et/ou
- réparer les cheveux vis à vis de l'oxydation.

Dans les produits de protection solaire, comprenant des filtres UV, par exemple de crèmes, laits, huiles, sprays solaires, les polyorganosiloxanes **A** selon l'invention peuvent avoir lui-même un effet de protection contre les effets des UV, sur la peau et/ou les cheveux. Il peut également avoir un effet d'amélioration de la protection d'autres agents, par exemple les filtres UV mentionnés ci-dessus, contre les effets des UV sur la peau et/ou sur les cheveux (synergie entre les polyorganosiloxanes **A** selon l'invention et d'autres agents). Les effets de protection contre les UV, peuvent également avoir un intérêt en ce qui concerne un maintien de l'aspect ou des performances de la composition dans le temps (moins de dégradations). Ainsi, les polyorganosiloxanes **A** selon l'invention peuvent éviter un jaunissement de la composition.

Bien entendu, le spécialiste veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Selon un mode de réalisation préféré, les compositions cosmétiques dépourvues de toxicité par contact cutané selon l'invention sont utilisées comme produit de soin et/ou d'hygiène d'une matière kératinique telle que la peau, le cuir chevelu, les cheveux, les cils, les sourcils, les ongles ou les muqueuses.

Selon un autre mode de réalisation préféré , la compositions cosmétique dépourvue de toxicité par contact cutané selon l'invention est caractérisée par le fait qu'elle constitue un produit capillaire rincé ou non rincé pour le lavage, la teinture, le soin, le conditionnement, le défrisage, le maintien de la coiffure ou la mise en forme permanente ou non des cheveux, une composition anti-solaire, un produit de soin bucco-dentaire ou un produit de maquillage.

Selon un autre mode de réalisation de l'invention, la composition cosmétique dépourvue de toxicité par contact cutané est caractérisée par le fait qu'elle est utilisée comme composition capillaire rincée, en particulier comme shampooing coiffant et/ou conditionneur, ou comme après-shampooing.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

Les compositions de l'invention peuvent être aussi utilisées comme produits de soin ou d'hygiène tels que des crèmes de protection, de traitement ou de soin pour le visage, pour les mains ou pour le corps, des laits corporels de protection ou de soin, des lotions, gels ou mousses pour le soin ou le nettoyage de la peau.

Les compositions de l'invention peuvent aussi être utilisées comme compositions antisolaires.

Les compositions peuvent également consister en des préparations solides constituant des savons ou des pains de nettoyage.

Les compositions peuvent être des produits pour le maquillage tels que des crèmes pour le visage, des fonds de teint, des mascaras, des eye-liners, des rouges à lèvres, des vernis à ongles.

Dans les compositions selon l'invention, les polyorganosiloxanes **A** peuvent représenter de 0,001 % à 10 % en poids, de préférence de 0,01 % à 5% en poids et plus particulièrement de 0,1 à 3% en poids par rapport au poids total de la composition finale.

Un autre objet de l'invention consiste en un procédé de traitement non-thérapeutique cosmétique de la peau, du cuir chevelu, des cheveux, des cils, des sourcils, des ongles ou des muqueuses, caractérisé par le fait qu'on applique sur le support kératinique une composition selon l'invention et telle que définie ci-dessus, selon la technique d'utilisation habituelle de cette composition.

Un autre objet de l'invention consiste en une utilisation en cosmétique d'une composition selon l'invention et telle définie ci-dessus pour le nettoyage et/ou le soin et/ou le conditionnement et/ou le coiffage des cheveux.

Un autre objet de l'invention consiste en une utilisation des polyorganosiloxanes A selon l'invention et tels que décrits ci-dessus pour le nettoyage et/ou le soin et/ou le conditionnement et/ou le coiffage des cheveux.

Enfin le dernier objet de l'invention consiste en un procédé de traitement cosmétique, comprenant l'application sur les cheveux d'une quantité efficace d'une composition cosmétique selon l'invention et telle que décrite ci-dessus.

Des exemples concrets, mais nullement limitatifs, illustrant l'invention vont maintenant être donnés.

### EXEMPLES

### EXEMPLE 1: Préparation de polyorganosiloxanes Ax dépourvus de toxicité par contact cutané selon l'invention

**Huile 1:** α,ω-bis(triméthylsilyl)-poly[diméthyl, méthyl [3-(2,2,6,6-tétraméthylpipéridin-4-yloxy)propyl]siloxane, (Huile Rhodorsil^{®} 21645 fournie par la société Bluestar Silicones France) - Viscosité: 10 000 mPa.s. - **Huile 2:** α,ω-bis(triméthylsilyl)-poly[diméthyl, méthyl [3-(2,2,6,6-tétraméthylpipéridin-4-yloxy)propyl]siloxane, (Huile Rhodorsil^{®} 21650 fournie par la société Bluestar Silicones France) - Viscosité: 90 000 mPa.s.

Pour les huiles **1** et **2**, avant le traitement selon l'invention, le taux des oligomères ayant une masse moléculaire M < 1000 Daltons est de l'ordre de 2 à 2,6% par rapport à la distribution moléculaire totale.

Procédé de "dévolatilisation poussée" selon l'invention: L'huile est chargée dans un réacteur, agitée mécaniquement et chauffé progressivement jusqu'à 170 °C (grâce à un fluide caloporteur qui amène de la chaleur à travers la paroi de l'appareil). Le taux d'oligomères (ayant une masse moléculaire M < 1000 Daltons) est réduit par une dévolatilisation poussée sous pressions réduite (4 mbar environ) pendant une duré suffisante de manière à ce qu'il soit inférieure à 1 %. Le taux est déterminé par une chromatographie par perméation de gel (GPC).

Les résultats sont consignés dans le Tableau II.

**Tableau II: Caractéristiques des huiles selon l'invention**

| | Matière première = Huile 1 (Rhodorsil^{®} 21645) | | | Matière première = Huile 2 (Rhodorsil^{®} 21650) | | |
|---|---|---|---|---|---|---|
| Polyorganosiloxanes **Ax** dépourvus de toxicité par contact cutané (à fonction pipéridinyle stériquement encombrée) selon l'invention (après dévolatilisation poussée) | **A1** | **A2** | **A3** | **A4** | **A5** | **A6** |
| Temps de dévolatilisation (h) à 4 mbar | 3 | 6 | 9 | 3 | 6 | 9 |
| Taux des oligomères ayant une masse moléculaire M < 1000 Daltons par rapport à la distribution moléculaire totale | 0,90 | 0,65 | 0,55 | 0,95 | 0,65 | 0,65 |
| Sensibilisation cutanée / Test LLNA | Négatif (non classée R43) | Négatif (non classée R43) | Négatif (non classée R43) | Négatif (non classée R43) | Négatif (non classée R43) | Négatif (non classée R43) |

### Résultats du test LLNA ("Local Lymph Node Assay") :

Ce test est réalisé pour chaque huile **A1** à **A6** selon l'invention et pour les huiles 1 et 2 (comparatifs) sur souris et se base sur l'induction de la prolifération de lymphocytes dans les ganglions sous-jacents au site d'application de la substance. Ainsi on évalue le pouvoir sensibilisant sur la peau (test conforme à la ligne directrice OCDE 429 ou de la méthode décrite dans l'annexe V de la directive 67/548/CEE en sa 29ème adaptation au progrès technique-2005/73/EC). Les résultats montrent que les huiles 1 et 2 sont classées "R43" (sensibilisantes) alors que les huiles A1 à A6 selon l'invention ne sont pas classées "R43" (non sensibilisante).

### Exemple 2: Préparation de compositions cosmétiques (shampooings):

On a réalisé des compositions de shampooing les unes conformes à l'invention: (compositions **I1, I2, I3, I4, I5 et I6**) et les autres comparatives (compositions **C1** et **C2**).

**Tableau III: Compositions des shampooings**

| Constituants | Invention | | | | | | Comparatif | |
|---|---|---|---|---|---|---|---|---|
| Lauryléthersulfate de sodium (C12/C14 à 70/30) à 2.2 moles d'oxyde d'éthylène | 11.2 g MA | 11.2 g MA | 11.2 g MA | 11.2 g MA | 11.2 g MA | 11.2 g MA | 11.2 g MA | 11.2 g MA |
| Cocoyl bétaïne à 30% de MA en solution aqueuse | 5,1 g MA | 5,1 g MA | 5,1 g MA | 5,1 g MA | 5,1 g MA | 5,1 g MA | 5,1 g MA | 5,1 g MA |
| Acide lauryl éther carboxylique (4.5 0E) | 3g MA | 3g MA | 3g MA | 3 g MA | 3g MA | 3g MA | 3gMA | 3g MA |
| Hydroxyéthylcellulose réticulée à l'épichlorhydrine et quaternisée par la triméthylamine, vendue sous la dénomination JR400 par la société AMERCHOL | 0,7 g | 0,7 g | 0,7 g | 0,7 g | 0,7 g | 0,7 g | 0,7 g | 0,7 g |
| Huiles polyroganosiloxanes à fonctions pipéridinyles stériquement encombrées (1,5 g MA) | **A1** | **A2** | **A3** | **A4** | **A5** | **A6** | **Huile 1** | **Huile 2** |
| Monoisopropanolamide d'acides de coprah | 2g | 2g | 2g | 2 g | 2g | 2g | 2g | 2g |
| Conservateurs, parfum | q.s | q.s | q.s | q.s | q.s | q.s | q.s | q.s |
| Hydroxyde de sodium q.s.p. | pH 5,3 | pH 5,3 | pH 5,3 | pH 5,3 | pH 5,3 | pH 5,3 | pH 5,3 | pH 5,3 |
| Eau déminéralisée q.s.p. | 100 g | 100 g | 100 g | 100 g | 100 g | 100 g | 100 g | 100g |
| Compositions | **I1** | **I2** | **I3** | **I4** | **I5** | **I6** | **C1** | **C2** |

| | | | | | | | | |
|---|---|---|---|---|---|---|---|---|
| "MA" signifie Matière Active. | | | | | | | | |

On effectue un shampooing en appliquant les compositions **I1, I2, I3, I4, 15 et I6** et les compositions **C1** et **C2** sur des chevelures de cheveux sensibilisés préalablement mouillés. On fait mousser le shampooing, on laisse pauser pendant 10 minutes puis on rince abondamment à l'eau. Un panel d'experts a évalué l'aspect des cheveux mouillés et séchés.

Résultats: sur cheveux mouillés, les compositions selon l'invention **I1, I2, I3, I4, I5 et I6** se comportent aussi bien que les compositions comparatives **C1** et **C2** racines plus décollées pour les cheveux traités avec la composition A selon l'invention. Sur cheveux séchés, le lissage visuel et au toucher est amélioré pour les cheveux traités avec la composition A selon l'invention.

### Exemple 3: Préparation de compositions cosmétiques (shampooings) comprenant 2,5 g de matière active d'huile polyorganosiloxane A à fonctions pipéridinyles stériquement encombrées selon l'invention

On prépare les mêmes compostions que celles décrites à l'Exemple 2 mais avec une quantité de 2,5 g de matière active en huile à fonction pipéridinyle (A1 à A6) au lieu de 1,5 g pour les compostions des essais comparatifs. En effet, du fait de l'innocuité des huiles A selon l'invention (non classé R43), il est à présent possible de formuler des compositions ayant une concentration en huile à fonction pipéridinyle (A1 à A6) supérieure au seuil des 2 g en matière active.

**Tableau IV: Compositions des shampooings**

| Constituants | Invention | | | | | | Comparatif | |
|---|---|---|---|---|---|---|---|---|
| Lauryléthersulfate de sodium (C12/C14 à 70/30) à 2.2 moles d'oxyde d'éthylène | 11.2 g MA | 11.2 g MA | 11.2 g MA | 11.2 g MA | 11.2 g MA | 11.2 g MA | 11.2 g MA | 11.2 g MA |
| Cocoyl bétaïne à 30% de MA en solution aqueuse | 5,1 g MA | 5,1 g MA | 5,1 g MA | 5,1 g MA | 5,1 g MA | 5,1 g MA | 5,1 g MA | 5,1 g MA |
| Acide lauryl éther carboxylique (4.5 0E) | 3g MA | 3g MA | 3g MA | 3gMA | 3gMA | 3 g MA | 3 g MA | 3g MA |
| Hydroxyéthylcellulose réticulée à l'épichlorhydrine et quaternisée par la triméthylamine, vendue sous la dénomination JR400 par la société AMERCHOL | 0,7 g | 0,7 g | 0,7 g | 0,7 g | 0,7 g | 0,7 g | 0,7 g | 0,7 g |
| Huiles (polyorganosiloxane à fonctions pipéridinyles stériquement encombrées (2,5 g MA) | **A1** | **A2** | **A3** | **A4** | **A5** | **A6** | **Huile 1** | **Huile 2** |
| Monoisopropanolamide d'acides de coprah | 2 g | 2g | 2g | 2g | 2g | 2g | 2g | 2g |
| Conservateurs, parfum | q.s | q.s | q.s | q.s | q.s | q.s | q.s | q.s |
| Hydroxyde de sodium q.s.p. | pH 5,3 | pH 5,3 | pH 5,3 | pH 5,3 | pH 5,3 | pH 5,3 | pH 5,3 | pH 5,3 |
| Eau déminéralisée q.s.p. | 100 g | 100 g | 100 g | 100 g | 100 g | 100 g | 100 g | 100 g |
| Compositions | **I7** | **I8** | **I9** | **I10** | **I11** | **I12** | **C1** | **C2** |

On effectue les mêmes tests qu'à l'Exemple 2.

**Résultats:** Sur cheveux séchés, le lissage visuel et au toucher est amélioré pour les cheveux traités avec les compositions **I7** à **I12** selon l'invention par rapport aux compositions des essais comparatifs (**C1** et **C2**).

## Revendications

1. Polyorganosiloxanes **A** dépourvus de toxicité par contact cutané, non classés comme sensibilisants cutanés «R43», comportant par molécule au moins un motif siloxyle substitué par au moins un groupement fonctionnel **V** comprenant au moins une fonction pipéridinyle stériquement encombrée lesdits polyorganosiloxanes **A** étant **caractérisés en ce qu'**après leur préparation on réduit le taux des monomères, oligomères et polymères ayant une masse moléculaire M<1000 Daltons à une valeur inférieure à 1% par rapport à la distribution moléculaire totale au moyen d'une technique permettant la séparation sélective des monomères, oligomères et polymères ayant une masse moléculaire **M** < 1000 Daltons.

2. Polyorganosiloxanes **A** dépourvus de toxicité par contact cutané selon la revendication 1 **caractérisés en ce que** la réduction du taux des monomères, oligomères et polymères ayant une masse moléculaire M < 1000 Daltons s'effectue par une dévolatilisation poussée sous une pression réduite comprise entre 3 et 20 mbar et à une température comprise entre 100 et 210°C jusqu'à ce que moins de 1% de la distribution moléculaire totale possède une masse moléculaire M< 1000 Daltons.

3. Polyorganosiloxanes **A** dépourvus de toxicité par contact cutané selon l'une quelconque des revendications précédentes **caractérisés en ce que** la réduction du taux des monomères, oligomères et polymères ayant une masse moléculaire M < 1000 Daltons s'effectue au moyen d'un réacteur batch, d'un évaporateur à film tombant, d'un évaporateur à film raclé, d'un évaporateur centrifuge ou d'un évaporateur continu avec flash.

4. Polyorganosiloxanes **A** dépourvus de toxicité par contact cutané selon l'une quelconque des revendications précédentes **caractérisés en ce qu'**ils comportent par molécule au moins un motif siloxyle substitué par au moins un groupement fonctionnel **V** directement lié à un atome de silicium, ledit groupement fonctionnel **V** étant un groupement à fonction(s) pipéridinyle(s) stériquement encombrée(s) choisi parmi le groupe constitué par:
a) des groupements de formule **(I)** : 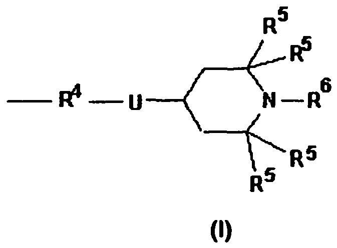 formule dans laquelle:
• Les radicaux R⁵, identiques ou différents, sont choisis parmi les radicaux alkyles linéaires ou ramifiés ayant 1 à 3 atomes de carbone et le radical phényle; et le radical R⁶ représente un radical hydrogène, le radical R⁵ ou l'atome O*;
• R⁴ est un radical hydrocarboné divalent choisi parmi le groupe constitué par :
- les radicaux alkylènes linéaires ou ramifiés, ayant 2 à 18 atomes de carbone;
- les radicaux alkylène-carbonyle dont la partie alkylène linéaire ou ramifiée, comporte 2 à 20 atomes de carbone;
- les radicaux alkylène-cyclohexylène dont la partie alkylène linéaire ou ramifiée, comporte 2 à 12 atomes de carbone et la partie cyclo-hexylène comporte un groupement OH et éventuellement 1 ou 2 radicaux alkyles ayant 1 à 4 atomes de carbone;
- les radicaux de formule R⁷-O-R⁷ dans laquelle les radicaux R⁷ identiques ou différents représentent des radicaux alkylènes ayant 1 à 12 atomes de carbone;
- les radicaux de formule R⁷-O-R⁷ dans laquelle les radicaux R⁷ ont les significations indiquées précédemment et l'un d'entre eux ou les deux sont substitués par un ou deux groupement(s) -OH;
- les radicaux de formule R⁷-COO-R⁷ dans laquelle les radicaux R⁷ ont les significations indiquées précédemment; et
- les radicaux de formule R⁸-O-R⁹-O-CO-R⁸ dans laquelle les radicaux R⁸ et R⁹ identiques ou différents, représentent des radicaux alkylènes ayant 2 à 12 atomes de carbone et le radical R⁹ est éventuellement substitué par un radical hydroxyle; et
• U représente -O- ou -N(R¹⁰)-, avec R¹⁰ étant un radical choisi parmi le groupe constitué par un atome d'hydrogène, un radical alkyle linéaire ou ramifié comportant 1 à 6 atomes de carbone et un radical divalent de formule (**II**) suivante : 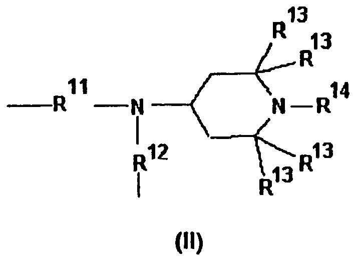
dans laquelle R¹² a la même signification que pour R⁴ indiqué précédemment, R¹¹ représente un radical divalent alkylène, linéaire ou ramifié, ayant de 1 à 12 atomes de carbone, l'un des liens valentiels (celui de R¹¹) étant relié à l'atome de -NR¹⁰-, l'autre (celui de R¹²) étant relié à un atome de silicium; les radicaux R¹³ sont identiques ou différents, choisis parmi les radicaux alkyles linéaires ou ramifiés ayant 1 à 3 atomes de carbone et le radical phényle; et le radical R¹⁴ représente un radical hydrogène, le radical R¹³ ou l'atome O*; et
b) des groupements de formule (**III**): 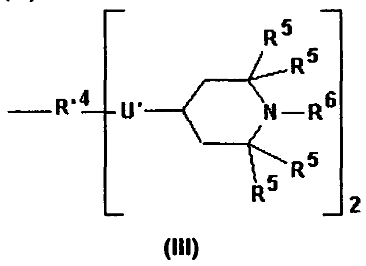 formule dans laquelle:
R'⁴ est choisi parmi le groupe constitué par :
- un radical trivalent de formule (**IV**) suivante: 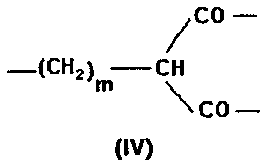 où m représente un nombre de 2 à 20, et
- un radical trivalent de formule **(VI) :** 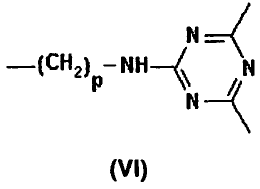 où p représente un nombre de 2 à 20;
U' représente -O- ou -N(R¹²)-, avec R¹² étant un radical choisi parmi le groupe constitué par un atome d'hydrogène et un radical alkyle linéaire ou ramifié comportant 1 à 6 atomes de carbone; et
R⁵ et R⁶ ont les mêmes significations que celles données à propos de la formule **(1);**
lesdits polyorganosiloxanes **A** sont **caractérisés en ce qu'**après leurs préparations on réduit le taux des monomères, oligomères et polymères ayant une masse moléculaire M < 1000 Daltons à une valeur inférieure à 1% par rapport à la distribution moléculaire totale au moyen d'une technique permettant la séparation sélective des monomères, oligomères et polymères ayant une masse moléculaire M < 1000 Daltons.

5. Polyorganosiloxanes **A** dépourvus de toxicité par contact cutané selon la revendication 1 ou 4 **caractérisés en ce que** le groupement fonctionnel **V** est un radical propyloxytétraméthylpipéridine de formule **(X)** suivante:

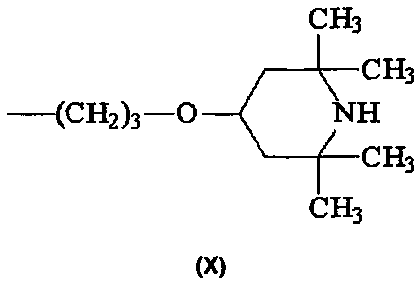

6. Polyorganosiloxanes **A** dépourvus de toxicité par contact cutané selon l'une des revendications précédentes **caractérisés en ce que** 0,8 à 4% des atomes de silicium sont substitués par un groupement fonctionnel **V** tel que décrit selon l'une quelconque des revendications précédentes.

7. Composition cosmétique et/ou dermatologique dépourvue de toxicité par contact cutané, non classée comme sensibilisant cutané « R43 », **caractérisée en ce qu'**elle comprend dans un milieu cosmétiquement acceptable au moins un polyorganosiloxane **A** tel que décrit selon l'une quelconque des revendications précédentes.

8. Composition cosmétique dépourvue de toxicité par contact cutané selon la revendication 7, **caractérisée par le fait qu'**elle se présente sous forme d'émulsion, de lotion, de gel, de dispersion vésiculaire, de pâte, de bâtonnet solide ou est conditionnée en aérosol et se présente sous forme de mousse ou de spray.

9. Composition cosmétique dépourvue de toxicité par contact cutané selon la revendication 7 ou 8, **caractérisée par le fait qu'**elle est utilisée comme produit de soin et/ou d'hygiène d'une matière kératinique telle que la peau, le cuir chevelu, les cheveux, les cils, les sourcils, les ongles ou les muqueuses.

10. Composition cosmétique dépourvue de toxicité par contact cutané selon la revendication 7, **caractérisée par le fait qu'**elle constitue un produit capillaire rincé ou non rincé pour le lavage, la teinture, le soin, le conditionnement, le défrisage, le maintien de la coiffure ou la mise en forme permanente ou non des cheveux, une composition anti-solaire, un produit de soin bucco-dentaire ou un produit de maquillage.

11. Composition cosmétique dépourvue de toxicité par contact cutané selon la revendication 7, **caractérisée par le fait qu'**elle est utilisée comme composition capillaire rincée, en particulier comme shampooing coiffant et/ou conditionneur, ou comme après-shampooing.

12. Procédé de traitement non-thérapeutique cosmétique d'une matière kératinique telle que la peau, le cuir chevelu, les cheveux, les cils, les sourcils, les ongles ou les muqueuses, **caractérisé par le fait qu'**on applique sur ladite matière kératinique une composition telle que définie selon l'une quelconque des revendications 7 à 11, selon la technique d'utilisation habituelle de cette composition.

13. Utilisation des polyorganosiloxanes **A** tels que définis selon l'une quelconque des revendications 1 à 6 pour le nettoyage et/ou le soin et/ou le conditionnement et/ou le coiffage des cheveux.

## Claims

1. Polyorganosiloxanes **A** devoid of toxicity upon skin contact, said polyorganosiloxanes not being classified as skin sensitizers "RA3", containing, per molecule, at least one siloxyl unit substituted with at least one functional group **V** comprising at least one sterically hindered piperidinyl functional group, said polyorganosiloxanes **A** being **characterized in that**, after their preparation, the level of monomers, oligomers and polymers having a molecular mass M < 1000 Daltons is reduced to a value less than 1% relative to the total molecular distribution by means of a technique allowing selective separation of the monomers, oligomers and polymers having a molecular mass **M** < 1000 Daltons.

2. Polyorganosiloxanes **A** devoid of toxicity upon skin contact according to Claim 1, **characterized in that** the reduction of the level of monomers, oligomers and polymers having a molecular mass M < 1000 Daltons is performed by extensive devolatilization under a reduced pressure between 3 and 20 mbar and a temperature between 100 and 210°C until less than 1% of the total molecular distribution has a molecular mass M < 1000 Daltons.

3. Polyorganosiloxanes **A** devoid of toxicity upon skin contact according to either of the preceding claims, **characterized in that** the reduction of the level of monomers, oligomers and polymers having a molecular mass M < 1000 Daltons is carried out by means of a batch reactor, a falling film evaporator, a scraped film evaporator, a centrifugal evaporator or a continuous flash evaporator.

4. Polyorganosiloxanes **A** devoid of toxicity upon skin contact according to any one of the preceding claims, **characterized in that** they contain, per molecule, at least one siloxyl unit substituted with at least one functional group **v** directly linked to a silicon atom, said functional group **V** being a group having one or more sterically hindered piperidinyl functional groups, chosen from the group consisting of:
a) groups of formula **(I):** 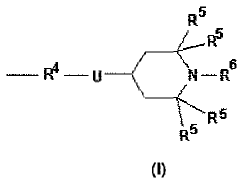 in which formula:
• the radicals R⁵, which are identical or different, are chosen from linear or branched alkyl radicals having 1 to 3 carbon atoms and the phenyl radical; and the radical R⁶ represents a hydrogen radical, the radical R⁵ or the O* atom;
• R⁴ is a divalent hydrocarbon radical chosen from the group consisting of:
- linear or branched alkylene radicals having 2 to 18 carbon atoms;
- alkylene-carbonyl radicals whose linear or branched alkylene part contains 2 to 20 carbon atoms;
- alkylene-cyclohexylene radicals whose linear or branched alkylene part contains 2 to 12 carbon atoms and the cyclohexylene part contains an OH group and optionally 1 or 2 alkyl radicals having 1 to 4 carbon atoms;
- the radicals of formula R⁷-O-R⁷ in which the radicals R⁷, which are identical or different, represent alkylene radicals having 1 to 12 carbon atoms;
- the radicals of formula R⁷-O-R⁷ in which the radicals R⁷ have the meanings indicated above and one of them or both are substituted with one or two -OH groups;
- the radicals of formula R⁷-COO-R⁷ in which the radicals R⁷ have the meanings indicated above; and
- the radicals of formula R⁸-O-R⁹-O-CO-R⁸ in which the radicals R⁸ and R⁹, which are identical or different, represent alkylene radicals having 2 to 12 carbon atoms and the radical R⁹ is optionally substituted with a hydroxyl radical; and
• U represents -O- or -N(R¹⁰)-, R¹⁰ being a radical chosen from the group consisting of a hydrogen atom, a linear or branched alkyl radical containing 1 to 6 carbon atoms and a divalent radical of the following formula **(II):** 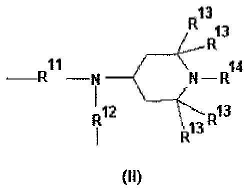
in which R¹² has the same meaning as for R⁴ indicated above, R¹¹ represents a linear or branched, divalent alkylene radical having from 1 to 12 carbon atoms, one of the valency bonds (that for R¹¹) being linked to the atom of -NR¹⁰-, the other (that for R¹²) being linked to a silicon atom; the radicals R¹³ are identical or different, chosen from the linear or branched alkyl radicals having 1 to 3 carbon atoms and the phenyl radical; and the radical R¹⁴ represents a hydrogen radical, the radical R¹³ or the O* atom; and
b) groups of formula **(III):** 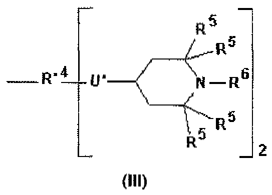 in which formula:
• R'⁴ is chosen from the group consisting of:
- a trivalent radical of the following formula **(IV)** : 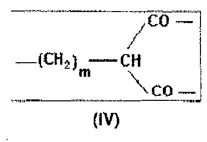
where m represents a number from 2 to 20, and
- a trivalent radical of formula (VI): 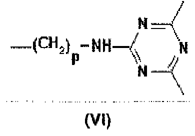
where p represents a number from 2 to 20,
• U' represents -O- or -N(R¹²)-, R¹² being a radical chosen from the group consisting of a hydrogen atom and a linear or branched alkyl radical containing 1 to 6 carbon atoms; and
• R⁵ and R⁶ have the same meanings as those given in the case of the formula **(I)**;
said polyorganosiloxanes **A** are **characterized in that** after their preparations, the level of monomers, oligomers and polymers having a molecular mass M < 1000 Daltons is reduced to a value less than 1% relative to the total molecular distribution by means of a technique allowing the selective separation of the monomers, oligomers and polymers having a molecular mass M < 1000 Daltons.

5. Polyorganosiloxanes **A** devoid of toxicity upon skin contact according to Claim 1 or 4, **characterized in that** the functional group **V** is a propyloxy-tetramethylpiperidine radical of the following formula (**X**):

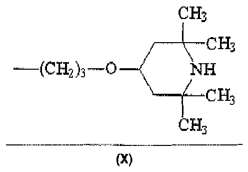

6. Polyorganosiloxanes **A** devoid of toxicity upon skin contact according to one of the preceding claims, **characterized in that** 0.8 to 4% of the silicon atoms are substituted by a functional group **V** as defined in any one of the preceding claims.

7. Cosmetic and/or dermatological composition devoid of toxicity upon skin contact, said composition not being classified as skin sensitizer "R43", **characterized in that** it comprises, in a cosmetically acceptable medium, at least one polyorganosiloxane **A** as defined in any one of the preceding claims.

8. Cosmetic composition devoid of toxicity upon skin contact according to Claim 7, **characterized in that** it is in the form of an emulsion, lotion, gel, vesicular dispersion, paste, solid stick or is packaged as an aerosol and is provided in the form of a mousse or a spray.

9. Cosmetic composition devoid of toxicity upon skin contact according to Claim 7 or 8, **characterized in that** it is used as a care and/or hygiene product for a keratin material such as the skin, the scalp, the hair, the eyelashes, the eyebrows, the nails or the mucous membranes.

10. Cosmetic composition devoid of toxicity upon skin contact according to Claim 7, **characterized in that** it consists of a rinse-off or leave-in hair product for washing, dyeing, caring for, conditioning, straightening, maintaining the hairstyle or permanent or nonpermanent shaping of the hair, an antisun composition, a buccodental care product or a makeup product.

11. Cosmetic composition devoid of toxicity upon skin contact according to Claim 7, **characterized in that** it is used as a rinse-off hair composition, in particular as a hairstyling and/or conditioning shampoo, or as a conditioner.

12. Method for the cosmetic nontherapeutic treatment of a keratin material such as the skin, the scalp, the hair, the eyelashes, the eyebrows, the nails or the mucous membranes, **characterized in that** a composition as defined in any one of Claims 7 to 11 will be applied to said keratin material, according to the customary technique for using this composition.

13. Use of the polyorganosiloxanes **A** as defined in any one of Claims 1 to 6, for cleansing and/or care and/or conditioning and/or styling of hair.

## Patentansprüche

1. Polyorganosiloxane A ohne Toxizität bei Hautkontakt, die nicht als hautsensibilisierender Stoff "R43" eingestuft sind, die pro Molekül mindestens ein Siloxyl-Motiv aufweisen, das mit mindestens einer funktionellen Gruppe V substituiert ist, die mindestens ein sterisch gehinderte Piperidinyl-Funktion trägt, wobei die Polyorganosiloxane A **dadurch gekennzeichnet sind, dass** man nach ihrer Herstellung den Anteil an Monomeren, Oligomeren und Polymeren mit einer Molekülmasse M < 1000 Dalton auf einen Wert unter 1%, bezogen auf die Gesamt-Molekülverteilung, mithilfe einer Technik verringert, die eine selektive Abtrennung der Monomere, Oligomere und Polymere mit einer Molekülmasse M < 1000 Dalton gestattet.

2. Polyorganosiloxane A ohne Toxizität bei Hautkontakt nach Anspruch 1, **dadurch gekennzeichnet, dass** die Verringerung des Anteils an Monomeren, Oligomeren und Polymeren mit einer Molekülmasse M < 1000 Dalton durch geförderte Entgasung unter einem verringertem Druck zwischen 3 und 20 mbar und bei einer Temperatur zwischen 100 und 210°C durchgeführt wird, bis weniger als 1% der Gesamt-Molekülverteilung eine Molekülmasse M < 1000 Dalton aufweist.

3. Polyorganosiloxane A ohne Toxizität bei Hautkontakt nach einem der vorhergehenden Ansprüche, **dadurch gekennzeichnet, dass** die Verringerung des Anteils an Monomeren, Oligomeren und Polymeren mit einer Molekülmasse M < 1000 Dalton mithilfe eines Batch-Reaktors, eines Fallfilmverdampfers, eines Dünnschichtverdampfers, eines Zentrifugalverdampfers oder eines kontinuierlichen Flash-Verdampfers durchgeführt wird.

4. Polyorganosiloxane A ohne Toxizität bei Hautkontakt nach einem der vorhergehenden Ansprüche, **dadurch gekennzeichnet, dass** sie pro Molekül mindestens ein Siloxyl-Motiv besitzen, das mit mindestens einer funktionellen Gruppe V substituiert ist, die direkt an ein Siliziumatom gebunden ist, wobei die funktionelle Gruppe V eine Gruppe mit (einer) sterisch gehinderten Piperidinyl-Funktion(en) ist, die aus der Gruppe ausgewählt wird, bestehend aus:
a) Gruppen der Formel (I): 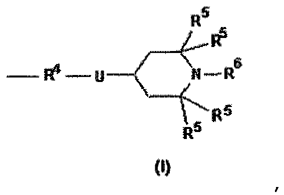 wobei in der Formel:
die Reste R⁵, die gleich oder verschieden sind, aus geraden oder verzweigten Alkylresten mit 1 bis 3 Kohlenstoffatomen und einem Phenylrest ausgewählt werden und der Rest R⁶ ein Wasserstoffatom, den Rest R⁵ oder das Atom O* bedeutet;
R⁴ ein zweiwertiger Kohlenwasserstoffrest ist, der aus der Gruppe ausgewählt wird, bestehend aus:
- geraden oder verzweigten Alkylenresten mit 2 bis 18 Kohlenstoffatomen;
- Alkylencarbonylresten, deren Alkylenanteil gerade oder verzweigt ist und 2 bis 20 Kohlenstoffatome trägt;
- Alkylencyclohexylenresten, deren Alkylenteil gerade oder verzweigt ist und 2 bis 12 Kohlenstoffatome trägt und deren Cyclohexylenanteil eine OH-Gruppe und gegebenenfalls 1 oder 2 Alkylreste mit 1 bis 4 Kohlenstoffatomen trägt;
- Resten der Formel R⁷-O-R⁷, wobei die Reste R⁷, die gleich oder verschieden sind, für Alkylenreste mit 1 bis 12 Kohlenstoffatomen stehen;
- Resten der Formel R⁷-O-R⁷, wobei die Reste R⁷ die vorstehend angegebenen Bedeutungen haben und einer von ihnen oder beide mit einer oder zwei OH-Gruppe(n) substituiert ist/sind;
- Resten der Formel R⁷-COO-R⁷, wobei die Reste R⁷ die vorstehend angegebenen Bedeutungen haben, und
- Resten der Formel R⁸-O-R⁹-O-CO-R⁸, wobei die Reste R⁸ und R⁹, die gleich oder verschieden sind, für Alkylenreste mit 2 bis 12 Kohlenstoffatomen stehen und der Rest R⁹ gegebenenfalls mit einem Hydroxylrest substituiert ist, und
U für -O- oder -N(R¹⁰)- steht, wobei R¹⁰ ein Rest aus der Gruppe ist, die aus einem Wasserstoffatom, einem geraden oder verzweigten Alkylrest mit 1 bis 6 Kohlenstoffatomen und einem zweiwertigen Rest der folgenden Formel (II) besteht: 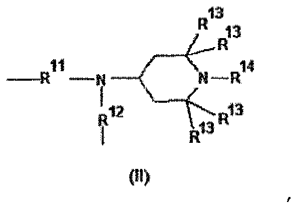 worin R¹² die gleiche Bedeutung hat, wie vorstehend für R⁴ angegeben, R¹¹ für einen geraden oder verzweigten zweiwertigen Alkylenrest mit 1 bis 12 Kohlenstoffatomen steht, wobei eine der Valenzbindungen (diejenige von R¹¹) an das Atom von -NR¹⁰- gebunden ist und die andere (diejenige von R¹²) an ein Siliziumatom gebunden ist, wobei die Reste R¹³ gleich oder verschieden sind und aus geraden oder verzweigten Alkylresten mit 1 bis 3 Kohlenstoffatomen und einem Phenylrest ausgewählt werden und der Rest R¹⁴ für ein Wasserstoffatom, den Rest R¹³ oder das Atom O- steht, und
b) Gruppen der Formel (III): 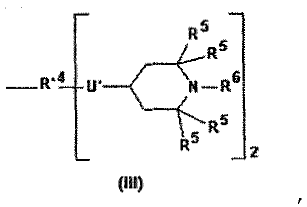 wobei in der Formel:
R'⁴ aus der Gruppe ausgewählt wird, bestehend aus:
- einem dreiwertigen Rest der folgenden Formel (IV) : 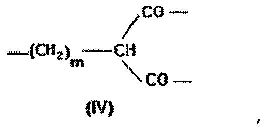 wobei m für eine Zahl von 2 bis 20 steht, und
- einem dreiwertigen Rest der Formel (VI): 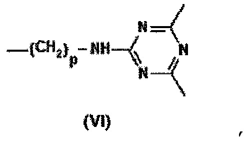
wobei p für eine Zahl von 2 bis 20 steht,
U' für -O- oder -N(R¹²)- steht, wobei R¹² für einen Rest aus der Gruppe steht, die aus einem Wasserstoffatom und einem geraden oder verzweigten Alkylrest mit 1 bis 6 Kohlenstoffatomen besteht, und
R⁵ und R⁶ die gleichen Bedeutungen haben, wie sie in Formel (I) angegeben sind;
wobei die Polyorganosiloxane A **dadurch gekennzeichnet sind, dass** man nach ihrer Herstellung den Anteil an Monomeren, Oligomeren und Polymeren mit einer Molekülmasse M < 1000 Dalton auf einen Wert unter 1%, bezogen auf die Gesamt-Molekülverteilung, mithilfe einer Technik verringert, die eine selektive Abtrennung der Monomere, Oligomere und Polymere mit einer Molekülmasse M < 1000 Dalton gestattet.

5. Polyorganosiloxane A ohne Toxizität bei Hautkontakt nach Anspruch 1 oder 4, **dadurch gekennzeichnet, dass** die funktionelle Gruppe V ein Propyloxytetramethylpiperidinrest der folgenden Formel (X) ist:

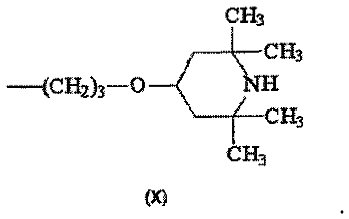

6. Polyorganosiloxane A ohne Toxizität bei Hautkontakt nach einem der vorhergehenden Ansprüche, **dadurch gekennzeichnet, dass** 0,8 bis 4% der Siliciumatome mit einer funktionellen Gruppe V nach einem der vorhergehenden Ansprüche substituiert sind.

7. Kosmetische und/oder dermatologische Zusammensetzung ohne Toxizität bei Hautkontakt, die nicht als hautsensibilisierender Stoff "R43" eingestuft ist, **dadurch gekennzeichnet, dass** sie in einem kosmetisch annehmbaren Medium mindestens ein Polyorganosiloxan A nach einem der vorhergehenden Ansprüche umfasst.

8. Kosmetische Zusammensetzung ohne Toxizität bei Hautkontakt nach Anspruch 7, **dadurch gekennzeichnet, dass** sie in Form einer Emulsion, einer Lotion, eines Gels, einer Vesikeldispersion, einer Paste, eines festen Stäbchens dargereicht wird oder zu einem Aerosol formuliert ist und in Form eines Schaums oder Sprays dargereicht wird.

9. Kosmetische Zusammensetzung ohne Toxizität bei Hautkontakt nach Anspruch 7 oder 8, **dadurch gekennzeichnet, dass** sie als Pflege- und/oder Hygieneprodukt für eine Keratinsubstanz, wie Haut, Kopfhaut, Haare, Wimpern, Augenbrauen, Nägel oder Schleimhäute verwendet wird.

10. Kosmetische Zusammensetzung ohne Toxizität bei Hautkontakt nach Anspruch 7, **dadurch gekennzeichnet, dass** sie ein ausgespültes oder nicht ausgespültes Haarprodukt zum Waschen, Färben, Pflegen, Konditionieren, Glätten, Aufrechterhalten der Frisur oder dauerhaften oder nicht dauerhaften Formen der Haare, eine Sonnenschutz-Zusammensetzung, ein bukkodentales Pflegeprodukt oder ein Schminkprodukt darstellt.

11. Kosmetische Zusammensetzung ohne Toxizität bei Hautkontakt nach Anspruch 7, **dadurch gekennzeichnet, dass** sie als ausgespülte Haarzusammensetzung, insbesondere als styling- und/oder Konditionierungsshampoo oder als Pflegespülung, verwendet wird.

12. Verfahren zur nicht-therapeutischen kosmetischen Behandlung einer Keratinsubstanz, wie Haut, Kopfhaut, Haare, Wimpern, Augenbrauen, Nägel oder Schleimhäute, **dadurch gekennzeichnet, dass** man auf die Keratinsubstanz eine Zusammensetzung nach einem der Ansprüche 7 bis 11 gemäß der üblichen Technik zur Verwendung dieser Zusammensetzung aufbringt.

13. Verwendung von Polyorganosiloxanen A nach einem der Ansprüche 1 bis 6 zur Reinigung und/oder Pflege und/oder zum Konditionieren und/oder Frisieren von Haaren.
